(19)

Europäisches Patentamt
European Patent Office
Office européen des brevets

(11) **EP 3 895 716 A1**

(12) **EUROPEAN PATENT APPLICATION**

(43) Date of publication:
**20.10.2021 Bulletin 2021/42**

(21) Application number: **20170256.0**

(22) Date of filing: **17.04.2020**

(51) Int Cl.:
*A61K 35/74* [(2015.01)]  *C12Q 1/04* [(2006.01)]
*C12Q 1/6869* [(2018.01)]  *C12Q 1/6806* [(2018.01)]
*C12Q 1/6876* [(2018.01)]

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Maat Pharma**
**69007 Lyon (FR)**

(72) Inventor: **The designation of the inventor has not yet been filed**

(74) Representative: **Santarelli**
**49, avenue des Champs-Elysées**
**75008 Paris (FR)**

(54) **FMT PERFORMANCE PREDICTION TEST TO GUIDE AND OPTIMIZE THERAPEUTIC MANAGEMENT OF GVHD PATIENTS**

(57) The present disclosure relates to a method for assessing whether a GVHD subject in need of a FMT can benefit from said FMT, by analysing the subject's microbiota and/or host parameters. Treatments for improving the status of patients identified as poor FMT responders are also provided, as well as materials and kits for performing the method according to the invention.

**Description**

**FIELD OF THE INVENTION**

The present invention pertains to the field of GVHD management. In particular, the present invention concerns the role of intestinal microbiota in GVHD and provides a method for determining if a patient is likely to benefit from a treatment aiming at modulating this microbiota, such as a fecal microbiota transplant (FMT), or if another treatment is necessary prior to the FMT to increase the patient's chances of success.

**BACKGROUND AND PRIOR ART**

*GVHD*

Graft versus host disease (GVHD) is a major complication of allogeneic hematopoietic stem cell transplantation (allo-HSCT) and consists of an immunologically mediated inflammatory reaction of donor immune T-cells against proteins, specifically human leukocyte antigens (HLAs), on host cells. Allo-HSCT is required for a curative intent in multiple hematologic disorders, varying from malignant diseases to genetic anaemias.
The rejection occurs when mismatch of minor histocompatibility antigens, or other reasons, trigger the donor's immune system to attack the recipient, causing unique inflammatory disease. GVHD does not occur after autologous HSCT (cells derived from the same patient).
GVHD have two main forms depending on the symptoms' timing:

- acute GVHD (aGVHD): a clinico-pathological syndrome that occurs within 100 days post HSCT (median onset is typically 21 to 25 days after transplantation); involving mostly three organs: the skin (>80% of patients with GVHD), gastrointestinal (GI) tract (50-55%) and liver (50%). Any one organ or combination of these organs may be affected.
- chronic GVHD (cGVHD) manifests with fibrotic skin disease, bronchiolitis, salivary and lacrimal gland disease, and eosinophilic fasciitis, and typically occurs more than 100 days post HSCT, often following acute GVHD.

*Current treatment of GVHD*

Management of GVHD is challenging. Immuno-suppression with corticosteroids forms the basis of first-line therapy in both acute and chronic GVHD, producing sustained responses in less than 50% of patients with aGVHD and 40-50% of patients with cGVHD, depending on initial disease severity.
For patients who do not respond well to steroids (steroid-refractory SR) or in whom steroids cannot be tapered (steroid-dependent SD), the prognosis is very poor.
A recent study described biomarkers to predict long-term outcome in steroid-resistant GVHD, including the levels of the suppressor of tumorigenicity-2 (ST2) and the regenerating-islet-derived protein 3-α (REG3α) after 1 week of systemic treatment (Major-Monfried *et al*., 2018).

*Importance of the patient's microbiota*

Patients undergoing allo-HSCT can be exposed to cytotoxic chemotherapy, total-body irradiation, immunosuppressors, and broad-spectrum antibiotics. These treatments cause dramatic alterations of the intestinal microbiota and varying degrees of damage to the intestinal mucosa, leading to breaches in host defenses.
Over the course of allo-HSCT, patients show profound shifts in microbial communities marked by a reduction of overall microbial diversity and richness, a disruption of beneficial bacteria that support host defenses (e.g., *Firmicutes*), and a rise in dominance of bacterial species usually subdominant, including some pathogens and pathobionts (e.g., *Clostridium difficile*, some *Enterobacteriaceae*) and multidrug-resistant (MDR) bacteria (Malard *et al.,* 2018), associated with subsequent bacteriemia and infectious complications (Taur *et al.,* 2012).
Current standard of care in oncology does not take into account the microbiota management, including its baseline status. However, several recent studies report that the gut microbiota is implicated in chemotherapy efficacy and toxicity through numerous mechanisms, including xenometabolism, immune interactions, and altered community structure. Without a functional microbiome, these treatments could be suboptimal (Iida *et al.,* 2013; Alexander *et al.,* 2017; Ma *et al.*, 2019).
In allo-HSCT patients, the diversity of the gut microbiota plays a key role in overall survival after allo-HSCT (Malard *et al.,* 2018), and in GVHD patient outcome (Taur *et al,* 2014, Peled *et al.*, 2020). Indeed, loss of microbiota diversity was observed to be associated with more pronounced gastrointestinal GVHD (Holler *et al.*, 2014). Several studies reported that disrupted microbiota (e.g. loss of diversity, domination by single taxa) are linked with poor patient outcomes as GVHD-related mortality, and insisted on the importance of the interaction between the microbiota and its host, and the opportunity to restore integrity to the intestinal microbiota (Malard *et al.*, 2018; Taur *et al.*, 2012; Taur *et al.*, 2014; Peled *et al*, 2020; Holler *et al.*, 2014; Golob *et al.*, 2017; Jenq *et al.*, 2015).

*FMT to restore gut microbiota in allo-HSCT patients*

Thus, strategies to manipulate the gut microbiota to suppress or decrease treatment-related complications in allo-HSCT patients were recently proposed, in addition to the standard of care armamentarium. Several case studies reported promising results of the use of Fecal Microbiota Transfer (FMT, also known as fecal microbiotherapy, defined as the administration of treated faeces from healthy donors via the upper or lower gastrointestinal route with the aim of restoring gut microbiota homeostasis) in the treatment of gastrointestinal aGVHD (Kakihana *et al.*, 2016; Spindelboeck *et al.*, 2017; Qi *et al.*, 2018 ; van Lier *et al.*, 2019; Shouval *et al.*, 2018).

However, given the small number of cases reported so far in this poor prognosis population of patients, it is difficult to identify which patient should and could actually benefit from such a FMT.

In absence of a definition of the targeted population, a patient could get the FMT although his/her clinical status will not allow the achievement of clinical response or his/her microbial intestine is not prepared to benefit from the fecal transplant product.

GVHD patient population is very fragile and with high mortality rate if an effective treatment is not put in place very quickly. Thus, treating patients who are not prepared to benefit from FMT treatments may lead to a loss of time and opportunity to be treated, within the context of critical illness and life-threatening emergency.

The present invention aims at fulfilling the unmet need for a FMT performance prediction test to guide and optimize therapeutic management of GVHD patients.

## SUMMARY OF THE INVENTION

The present invention pertains to a method for assessing whether a subject in need of a complementation of his/her gastrointestinal microbiota with live bacteria (e.g., FMT) can benefit from said complementation. This method is particularly advantageous for optimizing therapeutic management of GVHD patients, by distinguishing patients who can successfully receive such a complementation from those who will most likely not benefit from this treatment and need a conditioning treatment prior to receiving the live bacteria to improve the likelihood that these bacteria will engraft in their gut.

This method comprises:

a. measuring, in a gastrointestinal biological sample from said subject, the abundance of bacteria associated with a good prognosis, wherein said bacteria belong to at least one category selected from the group consisting of:

- Firmicutes phylum;
- Bacilli and Actinobacteria classes;
- Bacillales, Lactobacillales and Micrococcales orders;
- Staphylococcaceae, Lactobacillaceae, and Micrococcaceae families; and
- Staphylococcus, Lactobacillus, Melissococcus and Arthrobacter genera; and/or

b. measuring, in a gastrointestinal biological sample from said subject, the abundance of bacteria associated with a bad prognosis, wherein said bacteria belong to at least one category selected from the group consisting of:

- Bacteroidetes and Proteobacteria phyla;
- Bacteroidia class;
- Bacteroidales and Enterobacteriales orders;
- Bacteroidaceae, Porphyromonadaceae, Acidaminococcaceae, Lachnospiraceae, Ruminococcaceae, Clostridiaceae, Prevotellaceae and Erysipelotrichaceae families; and
- Bacteroides, Escherichia, Shigella, Ruminococcus, Faecalibacterium, Dorea, Coprococcus, Blautia, Alistipes, Subdoligranulum, Roseburia, Parabacteroides and Lachnospira genera; and

c. using the results obtained in step a and/or in step b and a calculation formula, calculating at least one score (#R) reflecting the likelihood that the subject's microbiota be significantly improved by said complementation; and

d. comparing each score obtained in step c to one or several reference values, and deducing whether the subject can successfully receive the complementation of his/her gastrointestinal microbiota with live bacteria or whether the subject needs a preparation treatment prior to said complementation.

According to other aspects of the invention, host parameters can be combined to the above microbiota parameters to predict the success or failure of FMT or other complementation treatment with live bacteria.

The present invention also pertains to the use of a FMT product for treating GvHD in a subject for whom the test was positive.

Another aspect of the invention is the use of conditioning treatments such as non-absorbable antibiotics targeting unfavorable bacteria and/or osmotic laxative treatments, to prepare the patient so that he/she can then benefit from FMT or another complementation treatment with live bacteria.

Materials and kits for performing the method according to the invention are also provided.

## LEGENDS TO THE FIGURES

Figure 1: clinical pathway according to the invention
Figure 2: HERACLES study design
Figure 3: HERACLES study, SR-aGvHD patients, BrayCurtis similarity vs IMP, OTU level
Figure 4: HERACLES study, SR-aGvHD patients, Evolution of the similarity with product compared to V1
Figure 5: HERACLES study, SR-aGvHD patients, MaaT indexes
Figure 6: EAP patients, BrayCurtis similarity vs IMP, OTU level
Figure 7: EAP patients, Butycore MaaT index
Figure 8: EAP patients, Health MaaT index
Figure 9: Blood citrulline
Figure 10: Indoxylsulfate
Figure 11: fecal zonulin
Figure 12: pre-albumin (blood)
Figure 13: total cholesterol
Figure 14: microbiota biomarkers of the gastrointestinal response at baseline
Figure 15: discriminant analysis results. A: effect size for each pre-selected taxon which has a significative stratifying effect. B: taxa grouped by taxonomic levels (P: Phylum, C: Class, O: Order, F: Family, G: Genus). Taxa with no significant effect on the prognostic signature are indicated in white.
Figure 16: additional microbiota biomarkers of the gastrointestinal response at baseline, with thresholds (abundances measured by 16S sequencing).

## DETAILED DESCRIPTION

The invention is particularly relevant in the clinical context of GVHD, especially in the context of steroid-refractory GVHD (SR-aGvHD). It is an optimization of the FMT treatment. Clinicians know that:

- on the one hand, treating a patient with a FMT that will poorly colonize the patient is not likely to be as efficient as wished. It can take time particularly precious for this patient population, and cost money although there is a low likelihood of treatment benefit. Indeed, some patients may need additional treatments prior to receiving the FMT, to increase the likelihood that the FMT efficiently modulates their microbiota and has clinical benefit.
- on the other hand, providing such "preparing treatments" to every patient would lead to treat patients who do not need such a treatment. This would at best lead to a loss of time, and at worse decrease the likelihood that these patients respond.

Hence, the present invention aims at providing a FMT performance prediction test to distinguish the patients who need a treatment prior to FMT to increase their chance of responding thereto, from those who do not need any such preparing treatment and can directly receive the FMT with a high chance of success.

In this context, two programs were set up by the Applicant to investigate the potential benefit of FMT in the GVHD population. The results of these programs, described in the experimental part which follows, led to a stratification tool to identify patients eligible to FMT.

The present invention thus pertains to a method for assessing whether a subject in need of a complementation of his/her gastrointestinal microbiota with live microorganisms can benefit from said complementation, comprising:

a. measuring, in a gastrointestinal biological sample from said subject, the abundance of bacteria associated with a good prognosis, wherein said bacteria belong to at least one category selected from the group consisting of:

- Firmicutes phylum;
- Bacilli and Actinobacteria classes;
- Bacillales, Lactobacillales and Micrococcales orders;
- Staphylococcaceae, Lactobacillaceae, and Micrococcaceae families; and
- Staphylococcus, Lactobacillus, Melissococcus and Arthrobacter genera; and/or

b. measuring, in a gastrointestinal biological sample from said subject, the abundance of bacteria associated with a bad prognosis, wherein said bacteria belong to at least one category selected from the group consisting of:

- Bacteroidetes and Proteobacteria phyla;
- Bacteroidia class;
- Bacteroidales and Enterobacteriales orders;
- Bacteroidaceae, Porphyromonadaceae, Acidaminococcaceae, Lachnospiraceae, Ruminococcaceae, Clostridiaceae, Prevotellaceae and Erysipelotrichaceae families; and
- Bacteroides, Escherichia, Shigella, Ruminococcus, Faecalibacterium, Dorea, Coprococcus, Blautia, Alistipes, Subdoligranulum, Roseburia, Parabacteroides and Lachnospira genera; and

c. using the results obtained in step a and/or in step b and a calculation formula, calculating at least one score (#R) reflecting the likelihood that the subject's microbiota be significantly improved by said complementation; and
d. comparing each score obtained in step c to one or several reference values, and deducing whether the subject can successfully receive the complementation of his/her gastrointestinal microbiota with live microorganisms or whether the subject needs a preparation treatment prior to said complementation.

As used herein, the term "comprise" or "include" is intended to mean that the compositions and methods include elements selected from the recited lists, without excluding other elements.

The singular forms "a" and "the" include plural references unless the context clearly dictates otherwise. Thus, e.g., reference to "a calculation formula" includes a plurality of calculation formulas.

As used herein, the term "good prognosis" means prognosis that the complementation will result in the engraftment of at least part of the administered live bacteria, whereas a "bad prognosis" means prognosis that the complementation will not result in a significant change in the microbiota composition.

When performing the claimed method, the skilled person can normalize the measured abundances, using any appropriate reference. Non-limiting examples of appropriate references include the total number of bacteria, the total number of bacteria + archea and, especially when the calculation in step c is a ratio between the levels of "good prognosis" and "bad prognosis" bacteria, any internal reference.

According to a particular embodiment of the above method, the following values are determined:

- a first value (#G) is determined in step a, and/or
- a second value (#B) is determined in step b,
- in step c, #R1=#G, #R2=1:#B and/or #R3=#G:#B, so that #R1, #R2 and/or #R3 is(are) above reference value(s) are indicative of a good prognosis and #R1, #R2 and/or #R3 inferior to reference value(s) are indicative of a bad prognosis.

In the above embodiment, #G is calculated with the measured abundances of the taxons selected for the bacteria associated with a good prognosis. For example, it can be the sum of the relative abundance of these taxons. Of course, this sum can be a weighted sum, to reflect each taxon's importance in the prognosis. For example, the skilled person can attribute a bigger weight to a taxon usually present in very low quantities but highly relevant for the prognosis, than the weight attributed to a taxon present in large quantities but poorly relevant in the prognosis.

The same reasoning applies to the calculation of #B.

The skilled person can also use, in step c, a formula more complex than the indicated ratios. The only condition is that the reference value(s) be adapted accordingly.

As used herein the "complementation of the subject's gastrointestinal microbiota with live microorganisms" designates administration of any composition comprising live microorganisms which can improve the subject's microbiota. Such a composition can comprise a pure culture of one single strain, a mix of several cultured strains and/or a complex material such as fecal material for performing Fecal Microbiota Transplantation (FMT).

According to a particular embodiment, the method according to the invention is performed for assessing whether a subject in need of a fecal microbiota transplant (FMT) can benefit from said transplant.

According to another particular embodiment, the subject suffers from a graft versus host disease (GvHD) following allogeneic hematopoietic stem cell transplantation (allo-HSCT).

The above method is particularly useful for assessing whether a subject who suffers from an acute, steroid-refractory graft versus host disease (SR-aGvHD) following allogeneic hematopoietic stem cell transplantation (allo-HSCT) can benefit from a complementation with live microorganisms (such as FMT), and/or in situations where the subject suffers from a GvHD with gastrointestinal impact.

When performing the above method, the skilled person is free to choose any combination of taxa amongst the different taxa indicated above as associated with good or bad prognosis. Taxa of same or different taxonomic levels can be combined. The skilled person can also combine these taxa with additional ones and, as already mentioned, the skilled person can use any relevant formula to calculate #G and/or #B values, respectively associated with good and bad prognosis. Non-limitative examples of formulas for performing the invention are indicated below (*n.b.*: the sums indicated below are to be understood as weighted sums of the indicated taxa - the skilled person can define the weight of each taxon to optimize the predictive value of the result):

(i) #G= Firmicutes, #B= Bacteroidetes and #R=#G:#B; and/or

(ii) #G= Firmicutes phylum excluding Acidaminococcaceae and Lachnospiraceae families, #B= Bacteroidetes and #R=#G:#B; and/or

(iii) #G= Firmicutes + Actinobacteria, #B= Bacteroidetes and #R=#G:#B; and/or

(iv) #G= Actinobacteria + Firmicutes excluding Acidaminococcaceae and Lachnospiraceae families, #B= Bacteroidetes and #R=#G:#B; and/or

(v) #G= Firmicutes, #B= Bacteroidetes + Proteobacteria and #R=#G:#B; and/or

(vi) #G= Firmicutes phylum excluding Acidaminococcaceae and Lachnospiraceae families, #B= Bacteroidetes + Proteobacteria and #R=#G:#B; and/or

(vii) #G= Firmicutes + Actinobacteria, #B= Bacteroidetes + Proteobacteria and #R=#G:#B; and/or

(viii) #G= Actinobacteria + Firmicutes excluding Acidaminococcaceae and Lachnospiraceae families, #B= Bacteroidetes + Proteobacteria and #R=#G:#B; and/or

(ix) #G= Firmicutes and #R=#G; and/or

(x) #G= Firmicutes phylum excluding Acidaminococcaceae and Lachnospiraceae families, and #R=#G; and/or

(xi) #G= Firmicutes + Actinobacteria and #R=#G; and/or

(xii) #G= Actinobacteria + Firmicutes excluding Acidaminococcaceae and Lachnospiraceae families and #R=#G; and/or

(xiii) #B= Bacteroidetes and #R=1:#B; and/or

(xiv) #B= Bacteroidetes + Proteobacteria and #R=1:#B; and/or

(xv) #G= Bacilli + optionally Actinobacteria, #B= Bacteroidia + optionally Gammaproteobacteria + optionally Negavicutes + optionally Clostridia and #R=#G:#B; and/or

(xvi) #G= Bacillales + Lactobacillales + Micrococcales, #B= Bacteroidales + Enterobacteriales + optionally Selenomonadales + optionally Clostridiales and #R=#G:#B; and/or

(xvii) #G= Staphylococcaceae + Lactobacillaceae + Micrococcaceae + optionally Enterococcaceae, #B= Bacteroidaceae + Porphyromonadaceae + Acidaminococcaceae + Lachnospiraceae + optionally Enterobacteriaceae and #R=#G:#B; and/or

(xviii) #G= Staphylococcus + Lactobacillus + Melissococcus + Arthrobacter, #B= Bacteroides + Escherichia + Shigella and #R=#G:#B.

(xix) #G= Bacilli + Micrococcales, #B= Bacteroidia + Enterobacteriales + Acidaminococcaceae + Lachnospiraceae and #R=#G:#B.

(xx) #B= Lachnospiraceae + Ruminococcaceae + Clostridiaceae, Prevotellaceae + Erysipelotrichaceae and #R=1:#B.

(xxi) #B= Bacteroides + Ruminococcus + Faecalibacterium + Dorea + Coprococcus + Blautia + Alistipes + Subdoligranulum + Roseburia + Parabacteroides + Lachnospira and #R=1:#B.

To perform the method of the invention, the skilled person can use any appropriate method for quantifying the bacteria. Non-limitative examples of such methods include quantitative PCR (qPCR), 16S sequencing, whole metagenomics sequencing, microarray, as well as culture and/or flow cytometry methods.

According to a particular embodiment of the invention, the abundances of the relevant taxa are measured by quantitative PCR. PCR techniques are well known and easily available and do not need a precise description. The PCR-based techniques are performed with amplification primers designed to be specific for the targets which are measured. The present invention hence also pertains to a set of primers suitable for performing the above method, i.e., a set of primers comprising primer pairs for amplifying sequences specific for each of the microorganism taxa to be detected in steps a and/or b of said method. Such a set of primers comprises a minimum of 4 primers, but it can comprise more primers, for example 6, 8, 10, 16, 20, 30, 40, 50, 60, 70, 80, 100, 200, 300, 500, 1000 or more primers. A kit of parts comprising such a set of primers and reactants for extracting bacterial DNA from a sample such as a rectal swab or stool sample is also part of the invention.

In another particular embodiment, the relative abundance of the selected species is assessed in step a and/or b by the use of a nucleic microarray. A "nucleic microarray" consists of different nucleic acid probes that are attached to a solid support, which can be a microchip, a glass slide or a microsphere-sized bead. Probes can be nucleic acids such as cDNAs ("cDNA microarray") or oligonucleotides ("oligonucleotide microarray"), and the oligonucleotides may be about 25 to about 60 base pairs or less in length. To determine the copy number of a target nucleic acid in a sample, this sample is labelled and contacted with the microarray in hybridization conditions so that complexes form between probe sequences attached to the microarray surface and target nucleic acids that are complementary thereto. The presence of labelled hybridized complexes is then detected. Many variants of the microarray hybridization technology are available to the skilled person.

A nucleic acid microarray designed to perform the method according to the invention is hence also part of the present invention. Such a nucleic acid microarray comprises nucleic acid probes specific for each of the bacterial taxa to be detected in step a and/or b of said method. The microarray according to the invention may further comprise at least one oligonucleotide for detecting at least one gene of at least one control bacterial species and/or any spiked-in control sequence. Preferably, the oligonucleotides are about 50 bases in length. Suitable microarray oligonucleotides may be designed, based on the genomic sequences specific for the relevant taxa, using any method of microarray oligonucleotide design known in the art. In particular, any available software developed for the design of microarray oligonucleotides may be used, such as, for instance, the OligoArray software, the GoArrays software, the Array Designer software, the Primer3 software, the mopo16s software or the Promide software, all known by the skilled in the art.

According to a further embodiment, determining the abundance of the relevant taxa in a sample obtained from the subject is performed using sequencing. Optionally, DNA is fragmented, for example by restriction nuclease or mechanical fragmentation prior to sequencing. Sequencing is done using any technique known in the state of the art, including sequencing by ligation, pyrosequencing, sequencing-by-synthesis, single-molecule sequencing or next-generation sequencing. Sequencing also includes PCR-Based techniques, such as for example emulsion PCR. A number of platforms are available for performing next-generation sequencing (NGS, also called "massive parallel DNA sequencing" or "high throughput DNA sequencing"), such as, but not limited to the Illumina Genome Analyzer platform, the Roche 454 platform, the ABI SOLiD platform, the Helicos single molecule sequencing platform, real-time sequencing using single polymerase molecules (Eid *et al.,* 2009), Ion Torrent sequencing (WO 2010/008480), PacBio sequencing (Rhoads *et al.*, 2015) and Oxford Nanopore sequencing (Clarke *et al.*, 2009).

According to yet another embodiment, the abundance of the relevant taxa in a sample obtained from the subject is measured through bacterial cultivation on selective media. For example, the fecal sample is diluted and then cultured under anaerobic conditions on a Petri dish with a medium selective for Firmicutes, and on another Petri dish with a medium selective for Bacteroidetes; bacteria are allowed to grow and then the colonies are counted to evaluate of the relative quantities of the 2 phyla.

The abundance of the relevant taxa in a sample obtained from the subject can also be measured by flow cytometry: for example, Firmicutes from a sample can be labeled with a fluorophore and Bacteroidetes with another fluorophore. The number of cells belonging to Firmicutes and Bacteroidetes is then assessed using a cytometer to measure the emitted fluorescence.

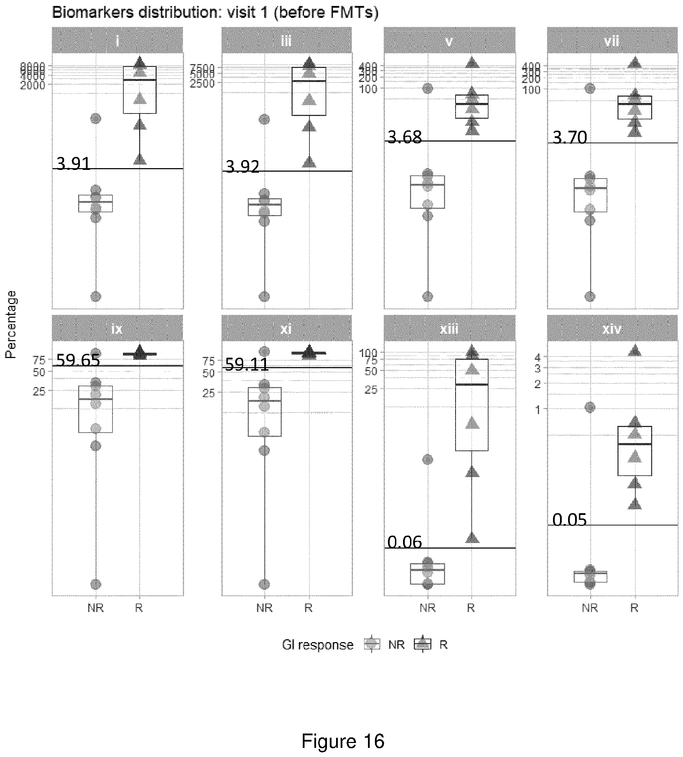

Examples of values that can be used as "reference values" in the frame of the invention are disclosed in the experimental part below (Example 5 and Figure 16). These values were obtained from a specific cohort, with bacterial abundances measured via 16S sequencing. Other examples of reference values are as follows:

- Using formula (xx) above, i.e., #B= Lachnospiraceae + Ruminococcaceae + Clostridiaceae + Prevotellaceae + Erysipelotrichaceae and #R=1:#B, a reference value is about 100, which means that #R>100 indicates that the subject is likely to benefit from the FMT.
- Using formula (xxi) above, i.e., #B= Bacteroides + Ruminococcus + Faecalibacterium + Dorea + Coprococcus + Blautia + Alistipes + Subdoligranulum + Roseburia + Parabacteroides + Lachnospira and #R=1:#B, a reference value is about 50, which means that #R>50 indicates that the subject is likely to benefit from the FMT.

Of course, the skilled artisan can adapt or refine these thresholds, depending on the technique used to measure the relative abundance of the microorganisms (for example, quantitative PCR, hybridization on a microarray or sequencing), the specific condition of the patient, the nature of the GI microbiota complementation with live microorganisms (e.g., FMT) to be administered, the nature of the sample used, the patient's food habits and other possible factors. More generally, the reference value to be considered when performing the above method is predetermined by measuring the relative abundance of the recited bacterial taxa in a representative cohort of individuals with a given condition, and whose response to a given treatment by GI microbiota complementation is known. The skilled person can also adjust the reference value(s) to favor the sensitivity and/or the specificity of the test.

According to a particular embodiment of the invention, the biological sample used in step a and/or b is a rectal swab or a feces sample.

Another aspect of the present invention relates to the prognostic value of certain host parameters (*i.e.*, parameters distinct from the microbiota composition) for assessing whether a subject in need of a complementation of his/her gastrointestinal microbiota with live microorganisms can benefit from said complementation. This aspect is supported by the results disclosed in Example 4 below, which show the prognostic relevance of the concentration of fecal zonulin and the blood concentrations of citrullin, prealbumin, cholesterol and indoxylsulfate. Two biomarkers, the suppressor of tumorigenicity-2 (ST2) and the regenerating-islet-derived protein 3-$\alpha$ (REG3$\alpha$), also known as MAGIC biomarkers, were previously described to predict long-term outcomes in steroid-resistant GVHD (non relapse mortality and overall survival) (Major-Monfried *et al.*, 2018). Preliminary results from the inventors with ST2 show that these markers at baseline are also predictive of the patient's response to FMT (data not shown).

The present invention thus also relates to a method for assessing whether a subject in need of a complementation of his/her gastrointestinal microbiota with live microorganisms can benefit from said complementation, comprising:

A. from at least one biological sample from the subject, measuring one, two, three, four, five, six or seven prognostic markers selected from the group consisting of the concentrations of cholesterol, indoxylsulfate, zonulin, citrullin, prealbumin, suppressor of tumorigenicity-2 (ST2) and regenerating-islet-derived protein 3-$\alpha$ (REG3$\alpha$);
B. comparing the values obtained in step a to reference values, wherein:

- zonulin concentration superior to a reference value;
- citrullin concentration superior to a reference value;
- prealbumin concentration superior to a reference value;
- cholesterol concentration superior to a reference value; and/or
- indoxylsulfate concentration inferior to a reference value;
- ST2 concentration inferior to a reference value; and/or
- REG3$\alpha$ concentration inferior to a reference value; are indicators of good prognosis.

The concentration of zonulin may be measured in any appropriate sample. According to a particular embodiment of this method, fecal zonulin concentration is measured in a rectal swab or a feces sample. Alternatively, blood zonulin concentration is measured, for example in serum or plasma.

In the above method, citrullin, prealbumin, cholesterol, indoxylsulfate, ST2 and/or REG3$\alpha$ can be measured from any appropriate biological sample from the patient. Non-limitative examples of suitable biological samples include blood, serum and plasma.

Of course, the skilled person can advantageously combine the methods described above, respectively based on the analysis of the subject's microbiota and on the analysis of certain host parameters, to increase the performance of the test. Methods combining both of these aspects are of course part of the present invention.

The present invention also pertains to the use of a composition of live microorganisms, preferably a FMT product, for treating GvHD in a subject for whom, based on the clinical patient profile and/or the result of a prediction test as above-described, the FMT is likely to succeed.

The prediction test is preferably used for at least SR-aGVHD patients. In addition, it can advantageously be applied in SD aGVHD, aGVHD with overlap syndrome or chronic GVHD patients, having already received at least one FMT and for whom FMT efficacy is not satisfactory, based on clinical symptoms and evaluation of FMT efficacy biomarkers (blood indoxyl sulfate, Butycore, Health index...).

As used herein, the term "treating" refers to any reduction or amelioration of the progression, severity, risk of relapse and/or duration of the symptoms of GvHD (especially GI symptoms).

By "FMT product" is herein meant any fecal microbial composition obtained (directly or indirectly) from a stool sample from (i) the patient him/herself prior to the treatment that led to allogeneic hematopoietic stem cell transplantation (ii) healthy individual(s), (iii) individual(s) exhibiting a microbiota profile most likely to be efficient for improving the patient's status, as well as to any such fecal microbial composition which has been enriched with one or several microbial strains. Several ways of conditioning fecal microbial material and conducting FMT have been described and are currently developed, and the skilled artisan is free to choose appropriate techniques for preparing the fecal microbial composition for use according to the invention, which can be freshly-prepared liquid, freeze-dried material or any other conditioning. Non-limitative examples of FMT products which can be used according to the present invention include FMT products described for example in WO2016/170285 or WO2019/171012, or products based on microbial culture of full or partial ecosystems containing at least 2 bacterial species. They can be administered either by enema or by the mean of a capsule for easier consumption (as described in WO2019/097030 for example), in which the product has been freeze-dried and powdered (as described in WO2017/103550 for example).

According to a particular embodiment, the subject treated by FMT according to the invention suffers from SR-aGvHD.

According to another particular embodiment, the subject treated by FMT according to the invention has gastrointestinal symptoms.

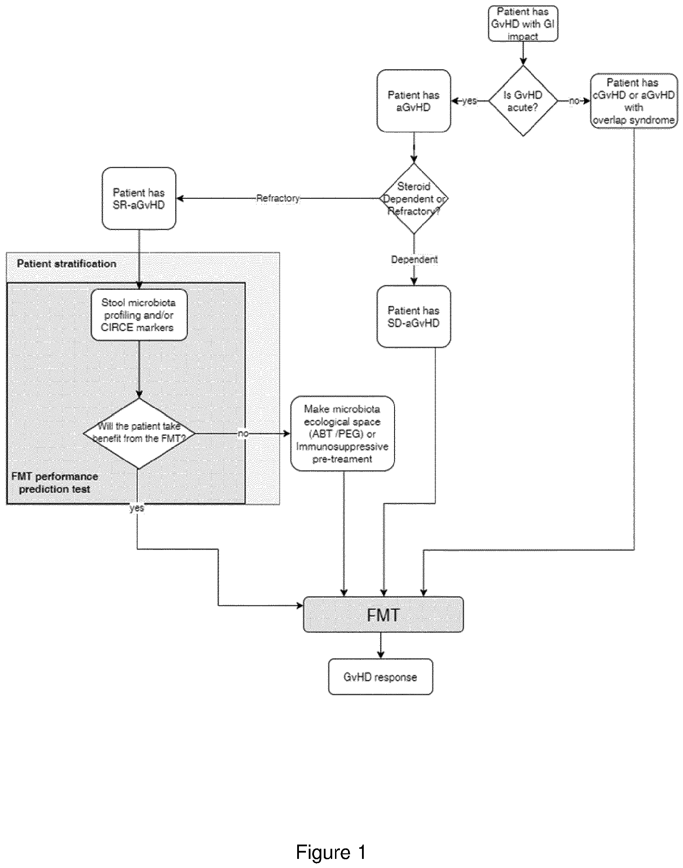

The FMT prediction test described above can be included in a broader clinical pathway for GVHD patients, described in Figure 1, that leverages the potential of FMT to treat such diseases. According to the GVHD subcategory, the patient is either oriented directly to the FMT therapy, or the potential effect of the FMT is tested. This is the "stratification" block that is built with the "FMT performance prediction Test". If the patient is identified by the above-described predictive test as less likely to benefit from FMT, he/she will be oriented to a treatment (e.g., antibiotherapy, PEG, ...) with the purpose to prepare his/her microbiota ecosystem beforehand the FMT. This GVHD clinical pathway (Figure 1) is also part of the present invention.

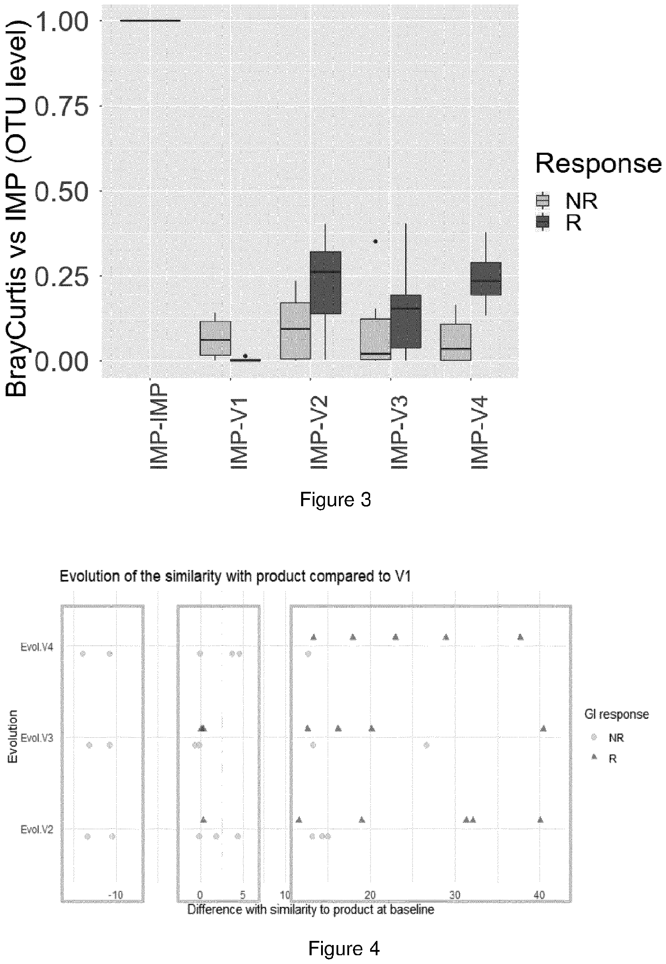

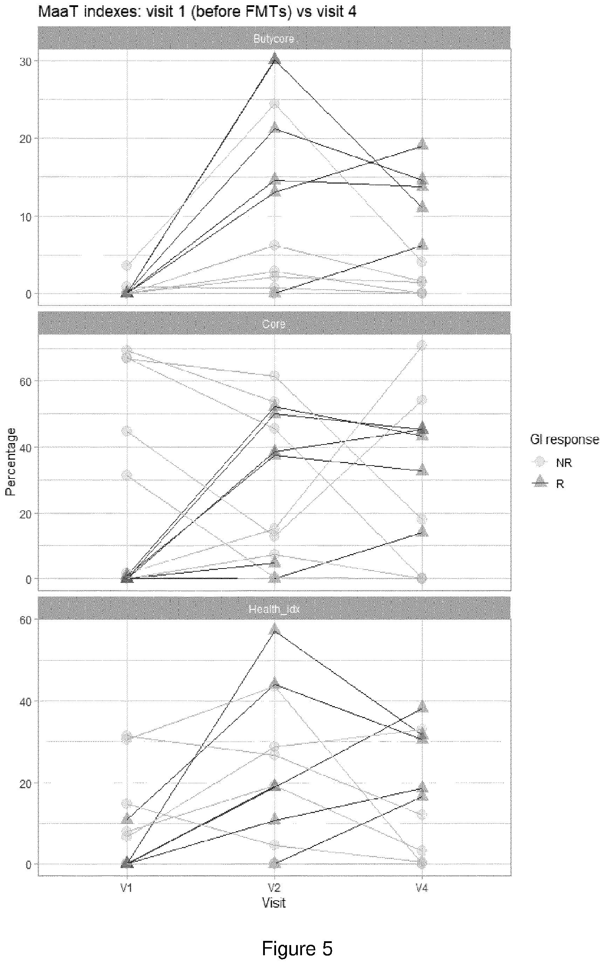

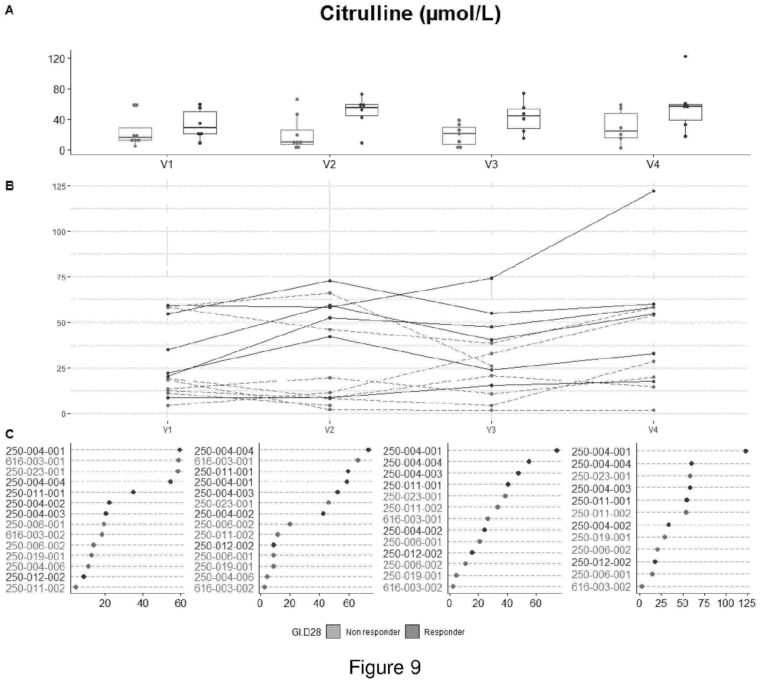

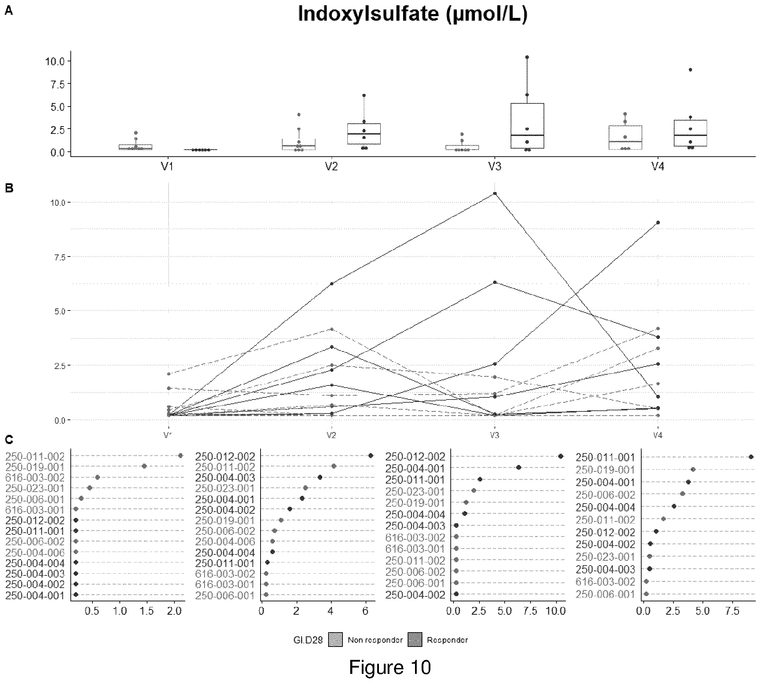

This GVHD clinical pathway can also comprise an additional step of monitoring the response to the FMT. Indeed, as shown in the experimental part below (Figure 4), an OTU BrayCurtis similarity with the FMT product that increases by more than 5 percentage points defines a block of patients who have a good GI response. The increasing Butycore or Health index (Figure 5) can also be used for monitoring purposes. Parameters measured in blood as citrullin (Figure 9) and Indoxyl-3-sulfate (Figure 10) concentrations, which increase for patients who have a good GI response as soon as the FMT occurred (visit 2), represent two alternative or additional means to monitor the response to the FMT.

The status of persons identified by the above-described predictive test as likely not to respond complementation of their gastrointestinal microbiota with live microorganisms can be significantly improved by a conditioning pre-treatment. Indeed, it is possible to induce a microbiota or host modification in patients identified as "non eligible" to FMT in order to make them eligible to FMT. For example, FMT pre-treatment with non-absorbable ABT targeting specific bacterial population, use of osmotic laxatives to reduce the burden of pathobionts in the gut, use of immunosuppressants to reduce the inflammatory state of the gut, or use of prebiotics to induce a shift in microbial communities - or any other process that addresses an ecological modification needs, can be adapted to the particular patient condition. Based on the markers described above, the skilled person can identify what ecological preparation would be the best for improving the product acceptability, and subsequently increase response likelihood. This preparation is preferably designed to at least eliminate bacteria who belong to the Bacteroidetes or the Proteobacteria phyla.

Non-limitative example of FMT pre-treatments include:

- non-absorbable antibiotics targeting specific bacterial population (e.g., vancomycin, gentamicin, colimycin, rifaximin, metronidazole, penicillin G and mixtures thereof),
- use of osmotic laxatives to reduce the burden of pathobionts in the gut
- use of prebiotics to induce a shift in microbial communities
- use of immunosuppressants to reduce the inflammatory state of the gut, and
- any other process that addresses an ecological modification needs.

According to a particular embodiment, the present invention pertains to the use of non-absorbable antibiotic selected from the group consisting of vancomycin, rifaximin, metronidazole, penicillin G and mixtures thereof, for treating a GVHD patient (with or without gastrointestinal symptoms) identified as likely not to respond to FMT. More particularly, the patient suffers from SR-aGvHD. According to this aspect of the invention, the antibiotic is administered prior to a FMT (or other treatment with live microorganisms).

According to another particular aspect of the invention, the patient receives an osmotic laxative in addition to or in replacement of the non-absorbable antibiotic targeting specific bacterial population. When both the osmotic laxative and the antibiotics are administered, the antibiotics are preferably administered prior to the laxative treatment.

As already mentioned, the present invention also relates to materials such as sets of primers and nucleic acids microarrays specifically designed to perform the above-described diagnostic/prognosis methods. Kits for companion diagnostic assay, comprising such materials, are thus also part of the present invention.

Other characteristics of the invention will also become apparent in the course of the description which follows of the biological assays which have been performed in the framework of the invention and which provide it with the required experimental support, without limiting its scope.

## EXAMPLES

The present invention is supported by the results of two programs set up to investigate the potential benefit of FMT in the GVHD population:

1) The ongoing phase 2 study (HERACLES) conducted by MaaT Pharma, that investigates the efficacy of a pooled FMT biotherapeutic, MaaT013 (described in WO2019/171012). We now expect that full ecosystem gut microbiota restoration with the MaaT013 biotherapeutic could be an effective treatment of gastrointestinal predominant SR-aGVHD, and thereby reduce the risk of life-threatening complications after allogeneic HSCT.

2) An Early Access Program (EAP) allowing the treatment of any GVHD situation (chronic/ acute, SD/SR).

These programs are further described below, as well as the materials and methods used to obtain the results described herein.

## HERACLES study

The population of the study consists of patients who developed a first episode of Grade III or IV aGVHD (= gastrointestinal stages 2 to 4) with gut predominance if other organs involved, resistant to a first line therapy with steroids, aged over 18 years old.

The primary objective of this study is to evaluate the gastrointestinal response at D28 through Complete Response (CR) and Very Good Partial Response (VGPR) of steroid refractory (SR) gastro-intestinal (GI) acute graft-versus-host disease (aGVHD) patients treated with allogeneic Fecal Microbiota Transfer (FMT).

The FMT product used during this study is the MaaT013 microbiota biotherapeutics manufactured by MaaT pharma. This product was obtained as described in WO2019/171012. It is referred in the figures as "IMP" for "investigational medicinal product".

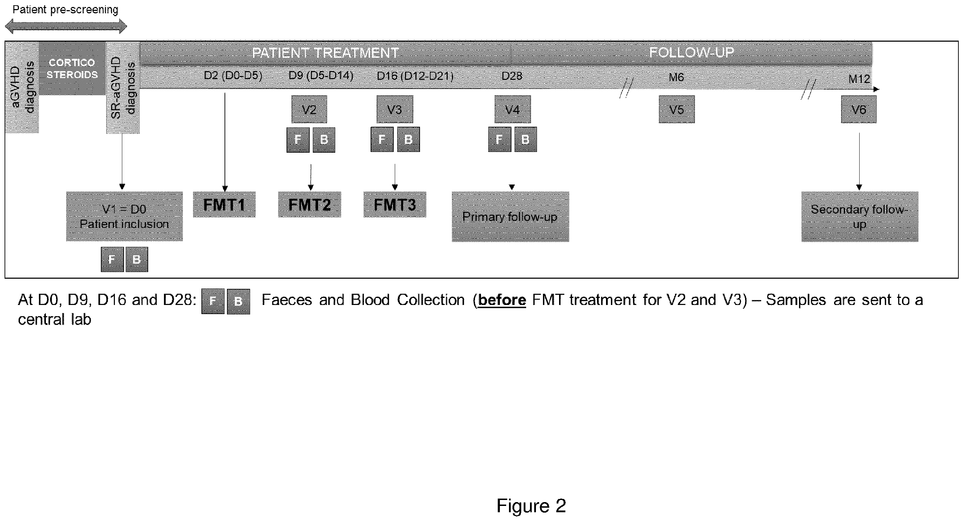

Figure 2 shows the design of the study.

### *Cohort of 15 patients*

   6 responders = R
   8 non-responders = NR
   1 patient died before V2 (not considered in the analyses)

### *Blood and fecal samples were collected at 4 visits*

   V1: before FMT
   V2: after FMT 1
   V3: after FMT 2
   V4: after FMT 3 (D28)

Some patients who failed to respond to FMT treatments received only 1 or 2 FMTs instead of 3 as planned.

The visit 1 (V1) stool collection allowed a microbiota profiling of the patient at baseline, *i.e.*, before receiving the FMT treatment. In the below analysis, patients are separated according to their gastro-intestinal (GI) response 28 days (D28) after FMT.

The evaluation of treatment responses was automatically calculated according to the following logic, based on GVHD grading and staging performed by the physicians at V4 (Day 28). The responses were calculated compared to GVHD evaluation at baseline (V1).

GI Response was considered as achieved in the following cases:

- Complete Response (CR): complete resolution of GI aGVHD manifestations, i.e. an improvement of the GI staging from any stage to 0
- Very Good Partial Response (VGPR): improvement of at least 2 stages in the severity of GI aGVHD, or improvement of the GI staging from 2 to 1, except improvement to stage 0
- Partial Response (PR): improvement of one stage in the severity of GI aGvHD, except improvement to stage 0 or improvement of the GI staging from 2 to 1

Patients were considered as non-responders in the following cases:

- Stable Disease (SD): persistence of the same stage of GI aGvHD
- Progressive Disease (PD): worsening of GI aGvHD of at least 1 stage
- If the patient receives additional systemic GVHD therapy before D28 (V4)
- If the patient dies before D28 (V4)

**Early Access Program (EAP)**

EAP was launched to answer the growing demands from physicians to treat GVHD patients with FMT.

All types of GVHD (acute/ chronic/overlap syndrome; SR or SD) treated with steroids associated with other lines of systemic treatment could be included in the EAP program, based on physician judgement regarding the medical need.

27 patients were treated with MaaT013.

- 19 patients had SR-aGVHD
- 8 patients:

  - 1 patient with SR-cGVHD
  - 4 patients with SD-aGVHD
  - 2 patients with SD-aGVHD with overlap syndrome
  - 1 patient with SR-aGVHD with overlap syndrome

The only data available for all of these patients are clinical GVHD responses.

For 4 patients, we obtained stool samples before each FMT treatment and at D28 (1 SR-cGVHD, 2 SD-aGVHD and 1 SD-aGVHD with overlap syndrome).

GI Response was considered as achieved in the following cases, 28 days after the first MaaT013 administration:

- Complete Response (CR): complete resolution of GI aGVHD manifestations, *i.e.*, an improvement of the GI staging from any stage to 0
- Very Good Partial Response (VGPR): improvement of at least 2 stages in the severity of GI aGVHD, or improvement of the GI staging from 2 to 1, except improvement to stage 0
- Partial Response (PR): improvement of one stage in the severity of GI aGvHD, except improvement to stage 0 or improvement of the GI staging from 2 to 1

Patients were considered as non-responders in the following cases:

- Stable Disease (SD): persistence of the same stage of GI aGvHD
- Progressive Disease (PD): worsening of GI aGvHD of at least 1 stage
- If the patient receives additional systemic GVHD therapy before D28
- If the patient died before D28

**Material and methods**

*16S rDNA sequencing and bioinformatics analysis of fecal microbiota*

16S rDNA sequencing was performed by Eurofins Genomics (Ebersberg, Germany). Genomic DNA was extracted using the NucleoSpin Soil kit (Machery Nagel). A sequencing library targeting the V3-V4 region of the 16S rRNA gene was constructed for each sample using the MyTaq HS-Mix 2X, Bioline, according to the manufacturer's instructions. Libraries were then pooled in an equimolar mixture and sequenced in paired-end (2x300 bp) MiSeq V3 runs, Illumina.

After amplicon merging using FLASH (Magoč *et al.,* 2011) and quality filtering using Trimmomatic (Bolger *et al.*, 2014), host sequence decontamination was performed with Bowtie2 (Langmead, Ben and Salzberg, 2013). Operational Taxonomic Unit (OTU) sequence clustering was performed with an identity threshold of 97% using VSEARCH (Rognes *et al.*, 2016) and taxonomic profiling was then performed with the Silva SSU database Release 128. For fair comparison, the sequence number of each sample was randomly normalized to the same sequencing depth i.e. 60000 amplicons per sample. Taxonomic and diversity analyses were performed with R Statistical Software (version 3.4.4).

*Host parameters (Blood, fecal)*

Albumin, Pre-albumin, total cholesterol were assessed on plasma with Cobas Integra 400+ / Kits ALBT2, PREA, CHOL2 from Roche Diagnostics respectively.

Indoxyl-3-sulfate and citrullin were assessed on plasma by Liquid-Chromatography - Mass Spectrometry.

Fecal zonulin was assessed on stool supernatants using ELISA kit (ELx800 reader/ IDK zonulin ref K5600) from Immundiagnostik AG.

**Example 1: FMT efficacy for patients with various GVHD (EAP program)**

Amongst the 19 patients with SR-aGVHD:

- 10 patients were considered as Non-responders to MaaT013
- 9 patients were considered as Responders to MaaT013.

Amongst the 8 patients with various GVHD except SR-aGVHD, all achieved GVHD response to MaaT013 (6 complete responses, 2 very good partial responses), which supports the direct orientation of these patients to a MaaT013 FMT.

**Example 2: Relationship between colonization performance of the FMT treatment and GI response (Heracles study)**

Figure 3 depicts the patient's microbiota similarity with the composition of the administrated FMT product, referred to as IMP. The higher the Bray Curtis value, the more similar to the product.
At V1, this similarity is low as expected because the FMT has not been administered yet. We also observe a difference between R and NR microbiotas. This may be related to the higher diversity of the NR patients' microbiota that increases the odds of having common OTUs with the product by chance.
At V2, V3 (after FMT pass 1 and FMT pass 2, respectively), the similarity with the product composition increases only for patients considered as responders at D28.
At V4, meaning D28, after the last FMT, this difference is the most pronounced (t-test, $p < 0.05$).
These data show that the patients' gut microbiotas do not react equally to the FMT. Some of them, i.e., the responders, have a microbiota composition that is modified after the FMT, and these modifications lead to a composition that gets closer to the administered product; some others, mostly the non-responders, do not get more similarity with the product.
Figure 4 illustrates the difference of the similarity percentage with the product (as described in Figure 3) between each of the V2, V3, V4 visits and the V1 visit. The range of evolution values can be divided into 3 groups: (1) group for which the evolution is negative, (2) group for which the evolution is low [close to 0, less than 5], and (3) group for which the difference is higher than 10. At V4, all responders are this third block, which includes also one of the non-responders (the lowest value in this block).
According to these evolution results, a similarity with the product that increases by a minimum of 5 percentage points is a good candidate to define a colonization by the product that impacts the patient's microbiota.
Conclusion: the similarity between the gut microbiota and the product microbiota is a FMT acceptance proxy, and the acceptance of the FMT is at least one of the factors that leverages patients' response.

*MaaT indexes*

Based on the combination of public and internal data, MaaT pharma has defined 3 indexes:

- *Core microbiome:* among public data and MaaT data for healthy subjects, it has been defined a common microbiome named core microbiome. The selected genera are the ones with >80% prevalence in the cohorts, and >0,1% median abundance per cohort. The genera list is: Ruminococcus, Faecalibacterium, Dorea, Coprococcus, Blautia, Alistipes, Bacteroides, Subdoligranulum, Roseburia, Parabacteroides, Lachnospira.
- *Health index:* an index of a healthy microbiome (containing bacteria families often associated with good health). This index is built by summing the relative abundances from those bacteria families: Lachnospiraceae, Ruminoccaceae, Clostridiaceae, Prevotellaceae, Erysipelotrichaceae.
- *Butycore:* sum or relative abundances of 15 butyrate producing genera: Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum and Butyrivibrio. The butycore can be interpreted as an anti-inflammatory potential microbiota marker.

As shown in Figure 5, the 3 MaaT defined indexes have increased between V1 and V4 in all responder patients. The Butycore, close to 0 at V1, is greater than 5% at V4 for all responders whereas it is below 5% for all non-responders. This metric is another good candidate for assessing the quality of the colonization performance.

**Example 3: Relationship between colonization performance of the FMT treatment and GI response (EAP program)**

For EAP patients with stool samples (1 SR-cGVHD, 2 SD-aGVHD and 1 SD-aGVHD with overlap syndrome - all were responders), according to data obtained during the EAP program, colonization performance metrics support the pattern outlined for SR-aGvHD data.

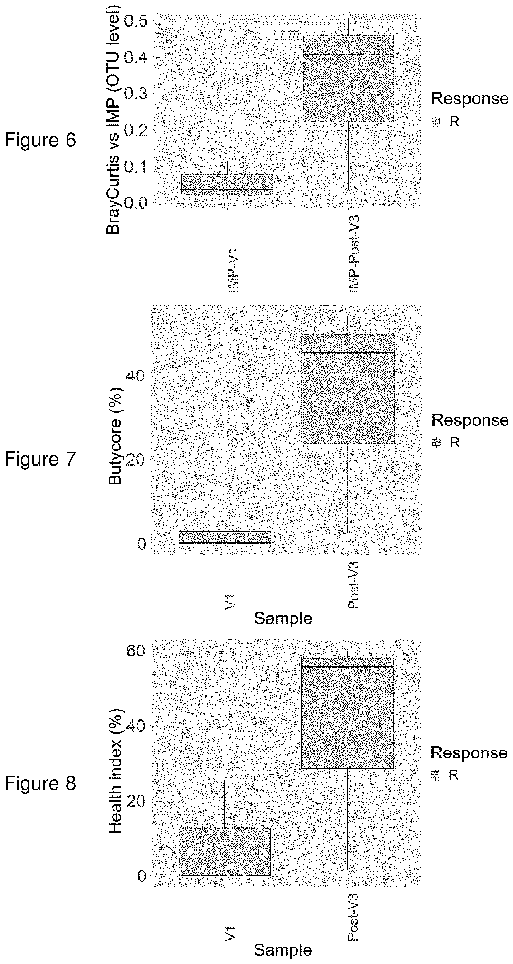

Figure 6 depicts the similarity of patients' microbiota with the composition of the administered IMP at V1 and Post-FMT3. The higher the Bray Curtis value, the more similar to the product. This demonstrates the engraftment of the microbiota of the IMP.

Figure 7 depicts the Butycore measured for the IMP, for patients at V1 and Post-V3. The Butycore has an increasing pattern from V1 to Post-V3, illustrating the quality of the colonization performance and the efficiency of FMT in microbiota reconstruction.

Figure 8 illustrates the Heath index measured for patients at V1 and Post-V3. The Heath index has an increasing pattern from V1 to Post-V3, illustrating the quality of the colonization performance and the efficiency of FMT in microbiota reconstruction.

Conclusion: These data show that the patients' gut microbiotas do not react equally to the FMT. They have a microbiota composition that is modified after the FMT, and the modifications lead to a more diverse composition that gets closer to the administered product for those patients who are all responders.

**Example 4: Evidences for host parameters markers (HERACLES study)**

***Citrulline***

Citrulline is an amino acid produced exclusively in small bowel enterocytes.

Because citrulline is not metabolized by the liver, its serum concentration correlates strongly with total functional enterocyte mass. It also correlates with age. Values can be influenced by renal function. Normal range of citrulline is 30-50 umol/L.

Citrullinemia is reduced in GI-aGVHD patients (Vokurka et al, Med Sci Monit 2013).

As shown in Figure 9, citrullin levels were higher in R patients after MaaT013 dosing (significant at V2 and V3).

The baseline value of citrullin also is a predictive biomarker, citrullin > 20 $\mu$mol/L indicating that the patient is likely to respond to the treatment.

***Indoxyl sulfate***

Indoxyl sulfate is a metabolite of l-tryptophan:
l-tryptophan $\rightarrow$ indole $\rightarrow$ indoxyl $\rightarrow$ indoxyl sulfate (IS)

Indole is produced from l-tryptophan in the human intestine via tryptophanase-expressing gastrointestinal bacteria. Indoxyl is produced from indole via enzyme-mediated hydroxylation in the liver. Subsequently, indoxyl is converted into indoxyl sulfate by sulfotransferase enzymes in the liver.

Urinary 3-IS levels predict outcome after HSCT and are associated with antibiotics. Low 3-IS levels within the first 10 days after HSCT are associated with significantly higher transplant-related mortality and overall lower survival 1 year after HSCT. Not only the diversity of the microbiome but its specific composition is indicative of urinary 3-IS. The majority of OTUs associated with high urinary 3-IS levels belong to the families of Lachnospiraceae (Eubacterium rectale) and Ruminococcaceae. Low 3IS were associated with members of the class of Bacilli (Weber et al., Blood 2015).

Factors associated with 3IS: lower IS concentrations in patients receiving ciprofloxacin/metronidazole compared with patients receiving rifaximin. Earlier systemic antibiotics treatment was also associated with low 3IS levels.

A decreased urinary excretion of 3-indoxyl sulfate (3-IS) is a marker of gut microbiota disruption and increased risk of developing gastrointestinal (GI) graft-versus-host-disease (Weber *et al.*, *supra*).

3-IS could not be assessed in urines of HERACLES patients (no urine collected) but we decided to test it in blood samples. Poor data are available in blood.

As shown in Figure 10, IS levels are a bit higher in R patients at V2, V3 and V4. IS levels seem to be increased after MaaT013 dosing, suggesting a beneficial impact of MaaT013 and may be a surrogate marker of engraftment.

***Fecal zonulin***

Human zonulin is a protein that increases permeability in the epithelial layer of the small intestine by reversibly modulating the intercellular tight junctions.

Among the several potential intestinal stimuli that can trigger zonulin release, small intestinal exposure to bacteria and gluten are the two triggers that have been identified so far. Enteric infections have been implicated in the pathogenesis of several pathological conditions, including allergic, autoimmune, and inflammatory diseases, by causing impairment of the intestinal barrier. Small intestines exposed to enteric bacteria secrete zonulin. This secretion is independent of the virulence of the microorganisms tested, occurred only on the luminal aspect of the bacteria-exposed small intestinal mucosa, and is followed by an increase in intestinal permeability coincident with the disengagement of the protein zonula occludens (ZO)-1 from the tight junctional complex. This zonulin-driven opening of the paracellular pathway may represent a defensive mechanism, which flushes out microorganisms, thereby contributing to the innate immune response of the host against bacterial colonization of the small intestine (Fasano, Clin Gastroenterol Hepatol 2012).

Fecal zonulin is elevated in Crohn's disease. Normal range is $61 \pm 46$ ng/ml (Malícková et al, Pract Lab Med 2017).

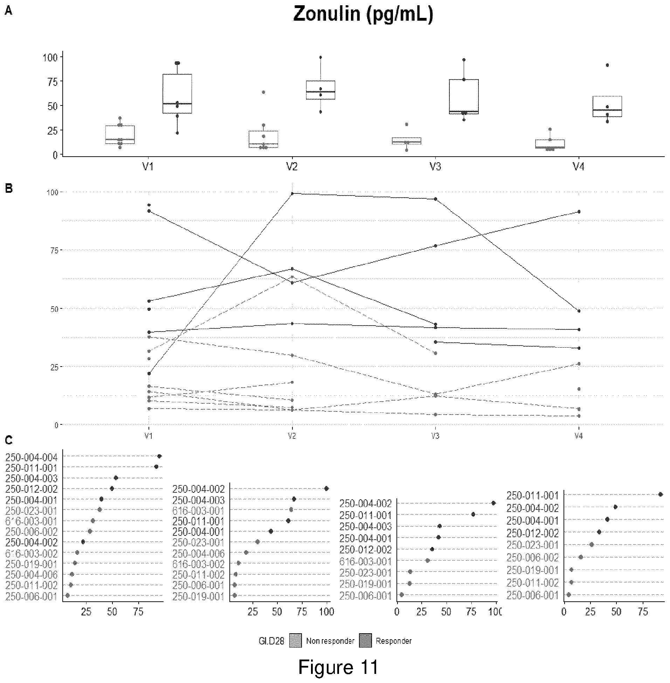

As illustrated in Figure 11, 3/4 responders measured at V2 have a slightly higher zonulin level than V1. At the cohort level, fecal zonulin is higher in responders than non-responders for all visits.

### Prealbumin

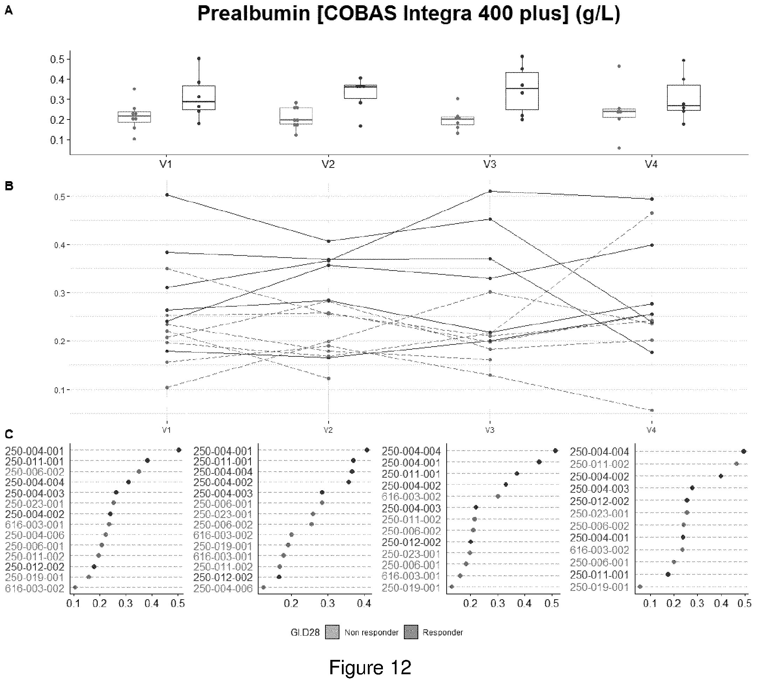

Figure 12 illustrates that prealbumin measurements are higher in responders than non-responders, more significantly at V2 and V3.

### Total cholesterol

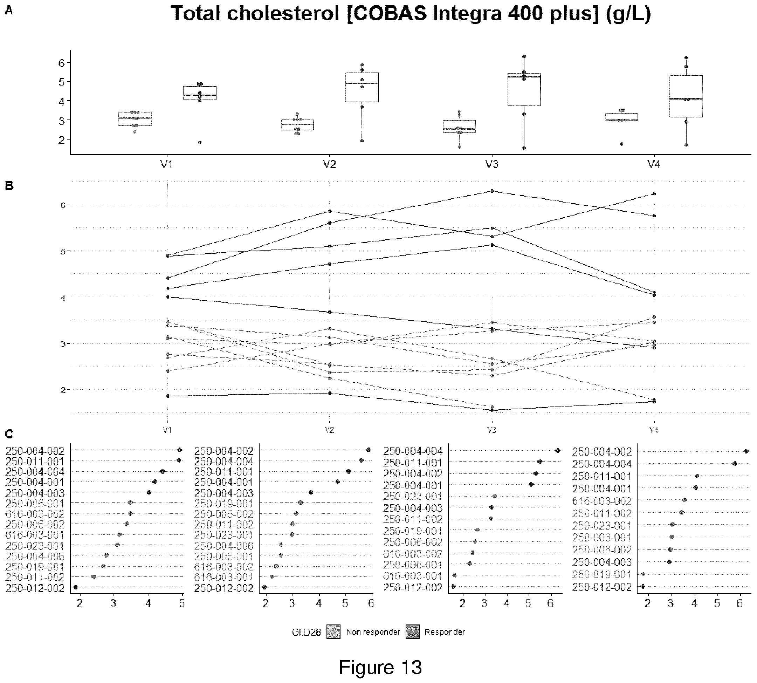

As shown in Figure 13, cholesterol is higher in responders (all visits). Subject 250-012-002, who has the lowest value for all visits, is the exception.

### Example 5: Evidences for microbiota markers predicting FMT response

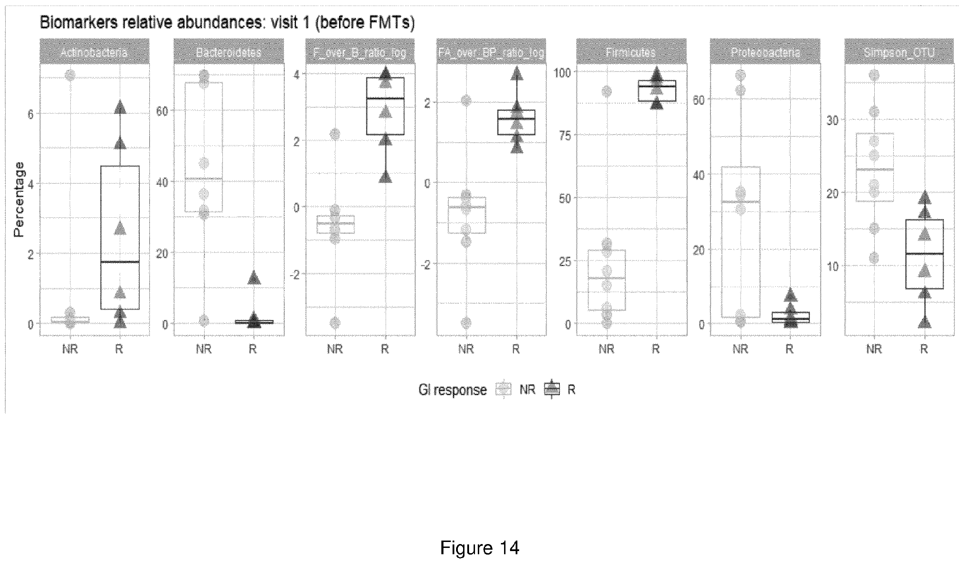

Figures 14 and 16 show a series of microbiota biomarkers, able to separate the population that responds (GI D28) from population that does not. The relative abundance of bacteria that belong to the *Firmicutes* phylum is a stratifying metric between responders (in dark grey) who have a higher relative abundance of *Firmicutes* (more than 80%), and non-responders (in light grey) who have a relative abundance of *Firmicutes* lower than 30% except for one whereas the FMT has never been administered (V1). The *Actinobacteria* also tend to be higher for most of responders (except for the highest value, 7%, which is reached by a non-responder). *Bacteroidetes* (less than 15% for R, more than 25% for most of NR) and *Proteobacteria* phylum (less than 10% for R, more than 25% for most of NR) have the opposite pattern: responders have lower values.

We also evaluated the ratio of good prognosis over bad prognosis phyla: (1) F_over_B_ratio_log which is the log10 transformation of the ratio *Firmicutes* over *Bacteroidetes,* and (2) FA_over_BP_ratio_log which is the log10 transformation of the ratio (*Firmicutes* + *Actinobacteria*) over (*Bacteroidetes* + *Proteobacteria*). For both of these ratios, all R have a value greater than 0, and all NR except 1/8 have a lower value (Figure 14).

Also, high alpha diversity indexes (represented here by the Simpson index at the OTU level) are likely to be bad prognosis biomarkers. All R have a Simpson index below 19%, which is not the case for 6/8 NR (Figure 14).

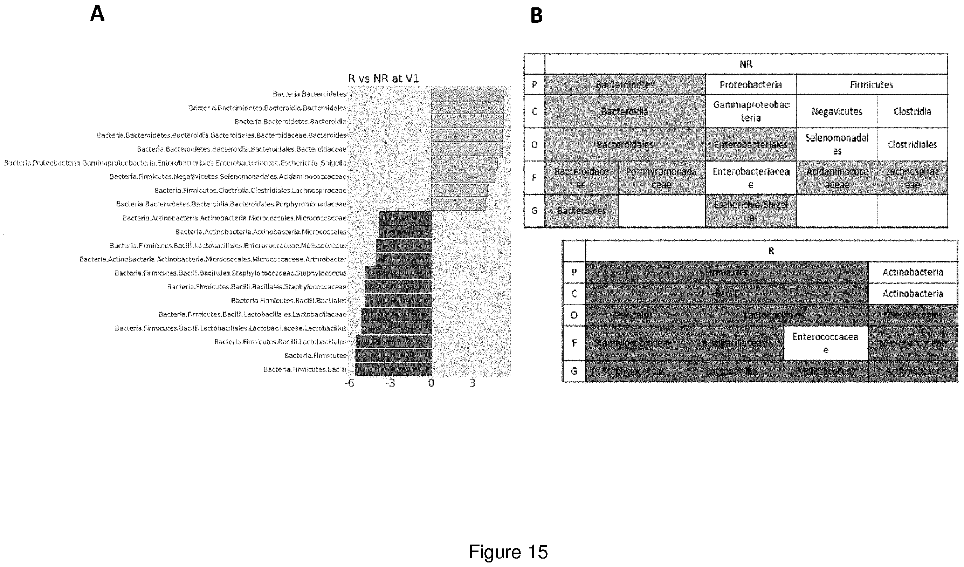

Figure 15 depicts the result of an analysis that selects important features which are informative in the separation of several groups (here 2 groups: responders and non-responders) and measures their quantitative effects. Figure 15A illustrates the size effect for each pre-selected taxon which has a significant stratifying effect. These taxa are presented in Figure 15B, grouped by taxonomic levels (P: Phylum, C: Class, O: Order, F: Family, G: Genus). Colored taxa are those which are useful for patients stratification, while the other indicated taxa have no effect on stratification.

Higher relative abundances of taxa highlighted in dark grey for a given patient before the FMT is predictive of a patient response, and higher level for the ones highlighted in light grey, or as illustrated in Figure 14, Health index, or core microbiota or diversity, is predictive of a non-response.

Figure 16 and in Table 1 below show non-limiting examples of formulas which can be used when performing the present invention.

Table 1: examples of formulas and associated thresholds (#R cut-offs) for performing the FMT performance prediction test in a SR-aGvHD patient with GI symptoms, with abundances of the recited taxa measured by 16S sequencing.

| Combination | #R cut-off |
| --- | --- |
| (i) #G= Firmicutes, #B= Bacteroidetes and #R=#G:#B; | 3,909 |
| (iii) #G= Firmicutes + Actinobacteria, #B= Bacteroidetes and #R=#G:#B; | 3,9225 |

(continued)

| Combination | #R cut-off |
|---|---|
| (v) #G= Firmicutes, #B= Bacteroidetes + Proteobacteria and #R=#G:#B; | 3,6848 |
| (vii) #G= Firmicutes + Actinobacteria, #B= Bacteroidetes + Proteobacteria and #R=#G:#B; | 3,7025 |
| (ix) #G= Firmicutes and #R=#G; | 59,651 |
| (xi) #G= Firmicutes + Actinobacteria and #R=#G; | 59,1075 |
| (xiii) #B= Bacteroidetes and #R=1:#B; | 0,057 |
| (xiv) #B= Bacteroidetes + Proteobacteria and #R=1:#B | 0,047 |

**Example 6: Relative abundance assessments using qPCR**
Quantitative PCR (qPCR, or real time PCR) will advantageously be used for performing the FMT performance prediction test according to the invention, for example using the protocol and primers described below.

*a. Choice of the qPCR technology*

Quantitative PCR (qPCR, or real time PCR) has several advantages because only a small amount of template DNA is required, it has a high sensitivity, a high-throughput processing, an affordable cost, and requires affordable equipment that is frequently found in laboratories (Bacchetti De Gregoris *et al.*, 2011).

*b. Protocol example*

Note: this protocol can evolve according to new primer design with updated 16S RNA gene sequences database.
DNA is extracted using a manufactured kit suitable for the extraction of DNA from fecal material, according to the manufacturer's instructions.
qPCR is performed in duplicates with a mix including SYBR and run on a multiwell (e.g. 96-well plate) real time PCR detection system.
Primers specific to the 16S rRNA region of bacterial taxa are used.
Each taxon-targeted qPCR (*i.e.*, each pair of primers) has to be carried out independently.
Examples of primers which can be used are described in Table 2 below.

Table 2: examples of primers which can be used to measure abundances of the recited taxa by qPCR when performing the FMT performance prediction test. Nucleotide symbols: R = A or G; Y = C or T; W = A or T; and S = C or G.

| Targeted taxa | Sequence (5'-3') | SEQ ID No | Reference |
|---|---|---|---|
| Firmicutes | F: GGAGYATGTGGTTTAATTCGAAGCA<br>R: AGCTGACGACAACCATGCAC | 1<br>2 | Guo *et al.*, 2008 |
| Bacteroidetes | F: GTTTAATTCGATGATACGCGAG<br>R: TTAASCCGACACCTCACGG | 3<br>4 | Yang *et al.*, 2015 |
| Bacteria | F: AGAGTTTGATCCTGGCTCAG<br>R: AAGGAGGTGWTCCARCC | 5<br>6 | Bacchetti De Gregoris *et al.*, 2011 |

PCR cycles parameters shall be optimized for this specific assay, as well as each primer pair efficiency. An example (Yang et al., 2015) provides these values:

Material: PCR system sequence detector with 2xFastStart SYBR green mix (Vazyme, Nanjing, China). qPCR mixtures contained 10μl of 2xFast-Start SYBR green with dye1, 0.5 μl of each forward and reverse primer (final concentration, 0.4μM), and 9μl of the DNAtemplate (equilibrated to 10 ng).
Annealing temperature of bacterial primers: 60°C.
Cycling conditions of denaturation: 95°C for 10 min, followed by 40 cycles of 95°C for 15 s and 60°C for 1 min.

Positive and negative controls shall be used.

*c. Relative abundance values for bacterial taxa*

Relative abundance values for bacterial taxa can be computed (Yang et *al.*, 2015) to total bacteria as follows:

$$ X = \frac{(\text{Eff.Bact})^{CT_{\text{bact}}}}{(\text{Eff.Spec})^{CT_{\text{spec}}}} \times 100 $$

where Eff.Bact (value between 1 and 2) is the calculated efficiency of the bacterial primers (2 = 100% and 1 = 0%), and Eff.Spec refers to the efficiency of the taxon-specific primers (Firmicutes, Bacteroidetes). $CT_{\text{bact}}$ and $CT_{\text{spec}}$ are the CT values registered by the thermocycler. "X" represents the percentage of 16S taxon-specific (e.g Firmicutes) copy number existing in a sample.

## REFERENCES

Alexander JL et al. Gut microbiota modulation of chemotherapy efficacy and toxicity. Nature Reviews - Gastroenterology and hepatology. 2017.

Bacchetti De Gregoris T et al. Improvement of phylum- and class-specific primers for real-time PCR quantification of bacterial taxa. J. Microbiol. Methods, vol. 86, no 3, p. 351-356, sept. 2011.

Bolger AM et al. Trimmomatic: A Flexible Trimmer for Illumina Sequence Data. Bioinformatics. 2014.

Clarke, J. et al. Continuous base identification for single-molecule nanopore DNA sequencing. Nat. Nanotechnol. 2009: 4, 265-270.

Eid, J et al. Real-time DNA sequencing from single polymerase molecules. Science 2009: 323, 133-138.

Golob JL et al. Stool Microbiota at Neutrophil Recovery Is Predictive for Severe Acute Graft vs Host Disease After Hematopoietic Cell Transplantation. Clinical Infectious Diseases. 2017.

Guo X et al. Development of a real-time PCR method for Firmicutes and Bacteroidetes in faeces and its application to quantify intestinal population of obese and lean pigs. Lett. Appl. Microbiol., vol. 47, no 5, p. 367-373, 2008.

Holler E, et al. Metagenomic analysis of the stool microbiome in patients receiving allogeneic stem cell transplantation: loss of diversity is associated with use of systemic antibiotics and more pronounced in gastrointestinal graft-versus-host disease. Biol Blood Marrow Transplant 2014.

Iida N et al. Commensal Bacteria Control Cancer Response to Therapy by Modulating the Tumor Microenvironment. Science 2013.

Jenq RR et al. Intestinal Blautia Is Associated with Reduced Death from Graft-versus-Host Disease. Biology of Blood and Marrow Transplantation. 2015.

Kakihana K, et al. Fecal microbiota transplantation for patients with steroid-resistant/dependent acute graft-versus-host disease of the gut. Blood 2016. Langmead B et al. Fast Gapped-Read Alignment with Bowtie 2. Nat Methods. 2012.

Ma et al. Gut Microbiota Shapes the Efficiency of Cancer Therapy. Front Microbiol. 2019.

Magoč T et al. FLASH: Fast Length Adjustment of Short Reads to Improve Genome Assemblies. Bioinformatics. 2011.

Major-Monfried H et al. MAGIC biomarkers predict long-term outcomes for steroid-resistant acute GVHD. Blood.

2018 Jun 21;131(25):2846-2855.

Malard Florent, Gasc Cyrielle, Plantamura Emilie, Doré Joël. High gastrointestinal microbial diversity and clinical outcome in graft-versus-host disease patients. Bone Marrow Transplantation 2018.

Malícková et al. Fecal zonulin is elevated in Crohn's disease and in cigarette smokers. Pract Lab Med. 2017 Sep 23;9:39-44.

Peled JU et al. Microbiota as Predictor of Mortality in Allogeneic Hematopoietic-Cell Transplantation. New England Journal of Medicine 2020.

Qi X. et al. Treating Steroid Refractory Intestinal Acute Graft-vs.-Host Disease With Fecal Microbiota Transplantation: A Pilot Study. Front Immunol. 2018.

Rhoads A and Au KF. PacBio Sequencing and Its Applications. Genomics Proteomics Bioinformatics. 2015 Oct;13(5):278-89.

Rognes T et al. VSEARCH: A Versatile Open Source Tool for Metagenomics. PeerJ. 2016.

Shouval R et al. Repeated Courses of Orally Administered Fecal Microbiota Transplantation for the Treatment of Steroid Resistant and Steroid Dependent Intestinal Acute Graft Vs. Host Disease: A Pilot Study (NCT 03214289). Blood 2018.

Spindelboeck W, et al. Repeated fecal microbiota transplantations attenuate diarrhea and lead to sustained changes in the fecal microbiota in acute, refractory gastrointestinal GVHD. Haematologica.2017.

Taur Y et al. Intestinal Domination and the Risk of Bacteremia in Patients Undergoing Allogeneic Hematopoietic Stem Cell Transplantation". Clin Infect Dis. 2012.

Taur Y et al. The effects of intestinal tract bacterial diversity on mortality following allogeneic hematopoietic stem cell transplantation". Blood 2014.

van Lier et al. Fecal Microbiota Transplantation Can Cure Steroid-Refractory Intestinal Graft-Versus-Host Disease. Biology of Blood and Marrow Transplantation. 2019

Vokurka et al. Serum citrulline levels as a marker of enterocyte function in patients after allogeneic hematopoietic stem cells transplantation - a pilot study. Med Sci Monit 2013; 19:81-85.

Weber D et al. Low urinary indoxyl sulfate levels early after transplantation reflect a disrupted microbiome and are associated with poor outcome. Blood. 2015.

Yang Y-W et al. Use of 16S rRNA Gene-Targeted Group-Specific Primers for Real-Time PCR Analysis of Predominant Bacteria in Mouse Feces, Appl. Environ. Microbiol., vol. 81, no 19, p. 6749-6756, oct. 2015.

SEQUENCE LISTING

<110>    MAAT PHARMA

<120>    FMT performance prediction test to guide and optimize therapeutic
          management of GVHD patients

<130>    B200114EPA

<160>    6

<170>    PatentIn version 3.5

<210>    1
<211>    25
<212>    DNA
<213>    Artificial Sequence

<220>
<223>    primer

<400>    1
ggagyatgtg gtttaattcg aagca                                                      25


<210>    2
<211>    20
<212>    DNA
<213>    Artificial Sequence

<220>
<223>    primer

<400>    2
agctgacgac aaccatgcac                                                            20


<210>    3
<211>    22
<212>    DNA
<213>    Artificial Sequence

<220>
<223>    probe

<400>    3
gtttaattcg atgatacgcg ag                                                         22


<210>    4
<211>    19
<212>    DNA
<213>    Artificial Sequence

<220>
<223>    primer

<400>    4
ttaasccgac acctcacgg                                                             19


<210>    5
<211>    20

```
<212>    DNA
<213>    Artificial Sequence

<220>
<223>    primer

<400>    5
agagtttgat cctggctcag                                              20


<210>    6
<211>    17
<212>    DNA
<213>    Artificial Sequence

<220>
<223>    primer

<400>    6
aaggaggtgw tccarcc                                                 17
```

**Claims**

1. A method for assessing whether a subject in need of a complementation of his/her gastrointestinal microbiota with live microorganisms can benefit from said complementation, comprising:

   a. measuring, in a gastrointestinal biological sample from said subject, the abundance of bacteria associated with a good prognosis, wherein said bacteria belong to at least one category selected from the group consisting of:

   - Firmicutes phylum;
   - Bacilli and Actinobacteria classes ;
   - Bacillales, Lactobacillales and Micrococcales orders;
   - Staphylococcaceae, Lactobacillaceae, and Micrococcaceae families ; and
   - Staphylococcus, Lactobacillus, Melissococcus and Arthrobacter genera ;
   and/or

   b. measuring, in a gastrointestinal biological sample from said subject, the abundance of bacteria associated with a bad prognosis, wherein said bacteria belong to at least one category selected from the group consisting of:

   - Bacteroidetes and Proteobacteria phyla;
   - Bacteroidia class;
   - Bacteroidales and Enterobacteriales orders;
   - Bacteroidaceae, Porphyromonadaceae, Acidaminococcaceae, Lachnospiraceae, Ruminococcaceae, Clostridiaceae, Prevotellaceae and Erysipelotrichaceae families; and
   - Bacteroides, Escherichia, Shigella, Ruminococcus, Faecalibacterium, Dorea, Coprococcus, Blautia, Alistipes, Subdoligranulum, Roseburia, Parabacteroides and Lachnospira genera; and

   c. using the results obtained in step a and/or in step b and a calculation formula, calculating at least one score (#R) reflecting the likelihood that the subject's microbiota be significantly improved by said complementation; and
   d. comparing each score obtained in step c to one or several reference values and deducing whether the subject can successfully receive the complementation of his/her gastrointestinal microbiota with live microorganisms or whether the subject needs a preparation treatment prior to said complementation.

2. The method of claim 1, wherein:

   - a first value (#G) is determined in step a, and/or
   - a second value (#B) is determined in step b,

- in step c, #R1 =#G, #R2=1:#B and/or #R3=#G:#B,

wherein if #R1, #R2 and/or #R3 is(are) superior to the reference value(s), the subject is likely to benefit from the complementation with live microorganisms, and if #R1, #R2 and/or #R3 is(are) inferior to the reference value(s), the subject needs a treatment prior to the complementation with live microorganisms for the microorganisms to successfully engraft in the subject's gut.

3. The method of claim 1 or claim 2, for assessing whether a subject in need of a fecal microbiota transplant (FMT) can benefit from said transplant.

4. The method according to any of claims 1 to 3, wherein the subject suffers from a graft versus host disease (GvHD) following allogeneic hematopoietic stem cell transplantation (allo-HSCT).

5. The method according to claim 4, wherein the subject suffers from an acute, steroid-refractory graft versus host disease (SR-aGvHD) following allogeneic hematopoietic stem cell transplantation (allo-HSCT).

6. The method according to claim 4 or claim 5, wherein the subject suffers from a GvHD with gastrointestinal impact.

7. The method of any of claims 2 to 6, wherein

(i) #G= Firmicutes, #B= Bacteroidetes and #R=#G:#B; and/or
(ii) #G= Firmicutes phylum excluding Acidaminococcaceae and Lachnospiraceae families, #B= Bacteroidetes and #R=#G:#B; and/or
(iii) #G= Firmicutes + Actinobacteria, #B= Bacteroidetes and #R=#G:#B; and/or
(iv) #G= Actinobacteria + Firmicutes excluding Acidaminococcaceae and Lachnospiraceae families, #B= Bacteroidetes and #R=#G:#B; and/or
(v) #G= Firmicutes, #B= Bacteroidetes + Proteobacteria and #R=#G:#B; and/or
(vi) #G= Firmicutes phylum excluding Acidaminococcaceae and Lachnospiraceae families, #B= Bacteroidetes + Proteobacteria and #R=#G:#B; and/or
(vii) #G= Firmicutes + Actinobacteria, #B= Bacteroidetes + Proteobacteria and #R=#G:#B; and/or
(viii) #G= Actinobacteria + Firmicutes excluding Acidaminococcaceae and Lachnospiraceae families, #B= Bacteroidetes + Proteobacteria and #R=#G:#B; and/or
(ix) #G= Firmicutes and #R=#G; and/or
(x) #G= Firmicutes phylum excluding Acidaminococcaceae and Lachnospiraceae families, and #R=#G; and/or
(xi) #G= Firmicutes + Actinobacteria and #R=#G; and/or
(xii) #G= Actinobacteria + Firmicutes excluding Acidaminococcaceae and Lachnospiraceae families and #R=#G; and/or
(xiii) #B= Bacteroidetes and #R=1:#B; and/or
(xiv) #B= Bacteroidetes + Proteobacteria and #R=1:#B; and/or
(xv) #G= Bacilli + optionally Actinobacteria, #B= Bacteroidia + optionally Gammaproteobacteria + optionally Negavicutes + optionally Clostridia and #R=#G:#B; and/or
(xvi) #G= Bacillales + Lactobacillales + Micrococcales, #B= Bacteroidales + Enterobacteriales + optionally Selenomonadales + optionally Clostridiales and #R=#G:#B; and/or
(xvii) #G= Staphylococcaceae + Lactobacillaceae + Micrococcaceae + optionally Enterococcaceae, #B= Bacteroidaceae + Porphyromonadaceae + Acidaminococcaceae + Lachnospiraceae + optionally Enterobacteriaceae and #R=#G:#B; and/or
(xviii) #G= Staphylococcus + Lactobacillus + Melissococcus + Arthrobacter, #B= Bacteroides + Escherichia + Shigella and #R=#G:#B.
(xix) #G= Bacilli + Micrococcales, #B= Bacteroidia + Enterobacteriales + Acidaminococcaceae + Lachnospiraceae and #R=#G:#B.
(xx) #B= Lachnospiraceae + Ruminococcaceae + Clostridiaceae, Prevotellaceae + Erysipelotrichaceae and #R=1:#B.
(xxi) #B= Bacteroides + Ruminococcus + Faecalibacterium + Dorea + Coprococcus + Blautia + Alistipes + Subdoligranulum + Roseburia + Parabacteroides + Lachnospira and #R=1:#B.

8. The method of any of claims 1 to 7, wherein said gastrointestinal biological sample is a rectal swab or a feces sample.

9. The method of any of claims 1 to 8, wherein bacteria are quantified by qPCR, 16S sequencing, whole metagenomics sequencing or by microarray.

10. The method of any of claims 1 to 9, wherein #B= Lachnospiraceae + Ruminococcaceae + Clostridiaceae + Prevotellaceae + Erysipelotrichaceae and #R=1 :#B>100 indicates that the subject is likely to benefit from the FMT.

11. The method of any of claims 1 to 9, wherein #B= Bacteroides + Ruminococcus + Faecalibacterium + Dorea + Coprococcus + Blautia, Alistipes + Subdoligranulum + Roseburia + Parabacteroides + Lachnospira and #R=1:#B>50 indicates that the subject is likely to benefit from the FMT.

12. The method of any of claims 1 to 11, further comprising:

   e. from at least one biological sample from the subject, measuring one, two, three, four or five prognostic markers selected from the group consisting of the concentrations of cholesterol, indoxylsulfate, fecal zonulin, citrullin, prealbumin, suppressor of tumorigenicity-2 (ST2) and regenerating-islet-derived protein 3-$\alpha$ (REG3$\alpha$);
   f. comparing the values obtained in step a to reference values, wherein:

   - fecal zonulin concentration superior to a reference value;
   - citrullin concentration superior to a reference value;
   - prealbumin concentration superior to a reference value;
   - cholesterol concentration superior to a reference value;
   - indoxylsulfate concentration inferior to a reference value;
   - ST2 concentration inferior to a reference value; and/or
   - REG3$\alpha$ concentration inferior to a reference value; are additional indicators of good prognosis.

13. The method of claim 12, wherein:

   - fecal zonulin concentration is measured in a rectal swab or a feces sample;
   - citrullin concentration is measured in blood plasma or serum;
   - prealbumin concentration is measured in blood plasma or serum;
   - cholesterol concentration is measured in blood plasma or serum;
   - indoxylsulfate concentration is measured in blood plasma or serum;
   - ST2 concentration is measured in blood plasma or serum; and/or
   - REG3$\alpha$ concentration is measured in blood plasma or serum.

14. A FMT product for use in the treatment of GvHD in a subject for whom the test by the method of any of claims 1 to 12 indicated that the FMT was likely to succeed.

15. A kit for performing the method of any of claims 1 to 13, comprising primers specific for the bacterial taxa for which the abundance is measured.

Figure 1

Patient pre-screening

| | | PATIENT TREATMENT | | | | FOLLOW-UP | |
|---|---|---|---|---|---|---|---|
| aGVHD diagnosis | CORTICO STEROIDS | SR-aGVHD diagnosis | D2 (D0-D5) | D9 (D5-D14) | D16 (D12-D21) | D28 | M6 | M12 |

V2 — F B

V3 — F B

V4 — F B

V5

V6

V1 = D0 Patient inclusion — F B

**FMT1**

**FMT2**

**FMT3**

Primary follow-up

Secondary follow-up

At D0, D9, D16 and D28: [F] [B] Faeces and Blood Collection (**before** FMT treatment for V2 and V3) – Samples are sent to a central lab

Figure 2

Figure 3

Figure 4

MaaT indexes: visit 1 (before FMTs) vs visit 4

Figure 5

Figure 6

Figure 7

Figure 8

Figure 9

Figure 10

Figure 11

EP 3 895 716 A1

Figure 12

Figure 13

Figure 14

Figure 15

Figure 16

Europäisches
Patentamt

European
Patent Office

Office européen
des brevets

# EUROPEAN SEARCH REPORT

Application Number

EP 20 17 0256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/143961 A1 (BERRY DAVID [US] ET AL) 26 May 2016 (2016-05-26) * [0396], [0457], [0461], [0545], [0553], [0594]-[596], ([0004], [0277], [0367]-[0368], [0541]-[0542], [0538]-([0539], [0776], [0020], [0037], [0051], [0199], [0202]-[0209], [0348], [0487]-[0490], [0287]-[0288], [0461], [0451]; examples 17-19, 26, 31, 33, 38-40, 53, 55, 80; table 1 * | 1-15 | INV.<br>A61K35/74<br>C12Q1/04<br>C12Q1/6869<br>C12Q1/6806<br>C12Q1/6876 |
| X | YUSUKE SHONO ET AL: "Gut microbiota injury in allogeneic haematopoietic stem cell transplantation", NATURE REVIEWS CANCER, vol. 18, no. 5, 16 February 2018 (2018-02-16), pages 283-295, XP055719222, ISSN: 1474-175X, DOI: 10.1038/nrc.2018.10 * abstract; figure 3 * * p. 284 col. 1, 1st , p. 287 col. 1, 2nd -p. 287 col. 2, 2nd , p. 290 col. 2, 1st , p. 292 col. 1, last -p. 292 col. 2, 1st , p. 284 col. 1, 1st , p. 284 col. 1, last -p.284 col. 2, last , p. 291 col. 2, last , p. 285 col. 2, 1st , p. 285 col. 1, 1st , p. 291 col. 2, 1st * | 1-15 | TECHNICAL FIELDS SEARCHED (IPC)<br><br>A61K<br>G01N<br>C12Q |

-/--

The present search report has been drawn up for all claims

| Place of search | Date of completion of the search | Examiner |
|---|---|---|
| Munich | 25 August 2020 | Landré, Julien |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 20 17 0256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHINSUKE KUSAKABE ET AL: "Pre- and post-serial metagenomic analysis of gut microbiota as a prognostic factor in patients undergoing haematopoietic stem cell transplantation", BRITISH JOURNAL OF HAEMATOLOGY, vol. 188, no. 3, 1 February 2020 (2020-02-01), pages 438-449, XP055719171, GB ISSN: 0007-1048, DOI: 10.1111/bjh.16205 * abstract; figures 1, 3-4 * * p. 439 col. 1, 2nd , p. 439 col. 1, last , p. 441 col. 1, last -p. 442 col. 1, 1st , p. 442 col. 1, last , p. 443 col. 2, 1st -2nd , p. 444 col. 1, last , p. 445 col. 1, 2nd -p. 445 col. 2, 2nd , p. 439 col. 2, 1st , p.447 col. 1, 2nd * | 1-15 | |
| X | E BIAGI ET AL: "Gut microbiota trajectory in pediatric patients undergoing hematopoietic SCT", BONE MARROW TRANSPLANTATION, vol. 50, no. 7, 20 April 2015 (2015-04-20), pages 992-998, XP055719347, GB ISSN: 0268-3369, DOI: 10.1038/bmt.2015.16 * p. 993 col. 1, 2nd , p. 993 col. 1, last -p. 993 col. 2, 1st , p. 993 col. 2, 4th -p.995 col. 1, 1st , p. 997 col. 1, last , (p. 995 col. 1, last -p. 997 col. 1, 1st , p. 997 col. 1, 2nd , p. 997 col. 2, 3rd , p. 997 col. 2, 2nd , p. 997 col. 2, last ; figures 1-4 * | 1,3,4,6, 8,9, 12-15 | TECHNICAL FIELDS SEARCHED (IPC) |

-/--

The present search report has been drawn up for all claims

| Place of search | Date of completion of the search | Examiner |
|---|---|---|
| Munich | 25 August 2020 | Landré, Julien |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

| | Europäisches Patentamt<br>European Patent Office<br>Office européen des brevets | **EUROPEAN SEARCH REPORT** | **Application Number**<br>EP 20 17 0256 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAKIHANA ET AL: "Fecal microbiota transplantation for patients with steroid-resistant acute graft-versus-host disease of the gut",<br>BLOOD,<br>vol. 128, no. 16,<br>20 October 2016 (2016-10-20), pages 2083-2088, XP055402621,<br>DOI: 10.1182/blood-2016-05-717652<br>* abstract; figure 1 *<br>* p. 2084 col. 1, last , p. 2085 col. 1, last , p. 2084 col. 2, 2nd -p. 2085 col. 1, last , p. 2087 col. 1, 1st -last * | 1-15 | |
| X | WO 2019/171012 A1 (MAAT PHARMA [FR]) 12 September 2019 (2019-09-12)<br>* p. 3, 1st , p. 4, 3rd , p. 8, penultimate , p. 8, 4th , p. 10, last , p. 15, last -p. 16, 1st , p. 17, 1st , p. 30, 1st , p. 43, last , p. 10, 1st -2nd ;<br>claims 13, 16; example 2 * | 14,15 | |

TECHNICAL FIELDS SEARCHED (IPC)

The present search report has been drawn up for all claims

| Place of search | Date of completion of the search | Examiner |
|---|---|---|
| Munich | 25 August 2020 | Landré, Julien |

EPO FORM 1503 03.82 (P04C01)

## ANNEX TO THE EUROPEAN SEARCH REPORT
## ON EUROPEAN PATENT APPLICATION NO.

EP 20 17 0256

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2020

| Patent document cited in search report | | Publication date | Patent family member(s) | | Publication date |
|---|---|---|---|---|---|
| US 2016143961 | A1 | 26-05-2016 | AU | 2015353425 A1 | 13-07-2017 |
| | | | CA | 2973223 A1 | 02-06-2016 |
| | | | CN | 108135944 A | 08-06-2018 |
| | | | EP | 3223834 A2 | 04-10-2017 |
| | | | HK | 1244683 A1 | 17-08-2018 |
| | | | US | 2016143961 A1 | 26-05-2016 |
| | | | US | 2016143962 A1 | 26-05-2016 |
| | | | US | 2016193258 A1 | 07-07-2016 |
| | | | US | 2016199424 A1 | 14-07-2016 |
| | | | US | 2016235792 A1 | 18-08-2016 |
| | | | US | 2016271188 A1 | 22-09-2016 |
| | | | US | 2018015130 A1 | 18-01-2018 |
| | | | US | 2018071344 A1 | 15-03-2018 |
| | | | US | 2018169153 A1 | 21-06-2018 |
| | | | US | 2019336543 A1 | 07-11-2019 |
| | | | US | 2020061129 A1 | 27-02-2020 |
| | | | WO | 2016086205 A2 | 02-06-2016 |
| | | | WO | 2016086206 A1 | 02-06-2016 |
| | | | WO | 2016086208 A1 | 02-06-2016 |
| | | | WO | 2016086209 A1 | 02-06-2016 |
| | | | WO | 2016086210 A1 | 02-06-2016 |
| WO 2019171012 | A1 | 12-09-2019 | FR | 3078627 A1 | 13-09-2019 |
| | | | WO | 2019171012 A1 | 12-09-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010008480 A
- WO 2016170285 A
- WO 2019171012 A
- WO 2019097030 A
- WO 2017103550 A

### Non-patent literature cited in the description

- **VOKURKA et al.** *Med Sci Monit,* 2013
- **WEBER et al.** *Blood,* 2015
- **FASANO.** *Clin Gastroenterol Hepatol,* 2012
- **MALÍČKOVÁ et al.** *Pract Lab Med,* 2017
- **ALEXANDER JL et al.** Gut microbiota modulation of chemotherapy efficacy and toxicity. *Nature Reviews - Gastroenterology and hepatology,* 2017
- **BACCHETTI DE GREGORIS T et al.** Improvement of phylum- and class-specific primers for real-time PCR quantification of bacterial taxa. *J. Microbiol. Methods,* September 2011, vol. 86 (3), 351-356
- **BOLGER AM et al.** Trimmomatic: A Flexible Trimmer for Illumina Sequence Data. *Bioinformatics,* 2014
- **CLARKE, J. et al.** Continuous base identification for single-molecule nanopore DNA sequencing. *Nat. Nanotechnol.,* 2009, vol. 4, 265-270
- **EID, J et al.** Real-time DNA sequencing from single polymerase molecules. *Science,* 2009, vol. 323, 133-138
- **GOLOB JL et al.** Stool Microbiota at Neutrophil Recovery Is Predictive for Severe Acute Graft vs Host Disease After Hematopoietic Cell Transplantation. *Clinical Infectious Diseases,* 2017
- **GUO X et al.** Development of a real-time PCR method for Firmicutes and Bacteroidetes in faeces and its application to quantify intestinal population of obese and lean pigs. *Lett. Appl. Microbiol.,* 2008, vol. 47 (5), 367-373
- **HOLLER E et al.** Metagenomic analysis of the stool microbiome in patients receiving allogeneic stem cell transplantation: loss of diversity is associated with use of systemic antibiotics and more pronounced in gastrointestinal graft-versus-host disease. *Biol Blood Marrow Transplant,* 2014
- **LIDA N et al.** Commensal Bacteria Control Cancer Response to Therapy by Modulating the Tumor Microenvironment. *Science,* 2013
- **JENQ RR et al.** Intestinal Blautia Is Associated with Reduced Death from Graft-versus-Host Disease. *Biology of Blood and Marrow Transplantation,* 2015
- **KAKIHANA K et al.** Fecal microbiota transplantation for patients with steroid-resistant/dependent acute graft-versus-host disease of the gut. *Blood,* 2016
- **LANGMEAD B et al.** Fast Gapped-Read Alignment with Bowtie 2. *Nat Methods.,* 2012
- **MA et al.** Gut Microbiota Shapes the Efficiency of Cancer Therapy. *Front Microbiol.,* 2019
- **MAGOČ T et al.** FLASH: Fast Length Adjustment of Short Reads to Improve Genome Assemblies. *Bioinformatics,* 2011
- **MAJOR-MONFRIED H et al.** MAGIC biomarkers predict long-term outcomes for steroid-resistant acute GVHD. *Blood,* 21 June 2018, vol. 131 (25), 2846-2855
- **MALARD FLORENT ; GASC CYRIELLE ; PLANTAMURA EMILIE ; DORÉ JOËL.** High gastrointestinal microbial diversity and clinical outcome in graft-versus-host disease patients. *Bone Marrow Transplantation,* 2018
- **MALÍČKOVÁ et al.** Fecal zonulin is elevated in Crohn's disease and in cigarette smokers. *Pract Lab Med.,* 23 September 2017, vol. 9, 39-44
- **PELED JU et al.** Microbiota as Predictor of Mortality in Allogeneic Hematopoietic-Cell Transplantation. *New England Journal of Medicine,* 2020
- **QI X et al.** Treating Steroid Refractory Intestinal Acute Graft-vs.-Host Disease With Fecal Microbiota Transplantation: A Pilot Study. *Front Immunol,* 2018
- **RHOADS A ; AU KF.** PacBio Sequencing and Its Applications. *Genomics Proteomics Bioinformatics,* October 2015, vol. 13 (5), 278-89
- **ROGNES T et al.** VSEARCH: A Versatile Open Source Tool for Metagenomics. *PeerJ,* 2016

- **SHOUVAL R et al.** Repeated Courses of Orally Administered Fecal Microbiota Transplantation for the Treatment of Steroid Resistant and Steroid Dependent Intestinal Acute Graft Vs. Host Disease: A Pilot Study (NCT 03214289). *Blood,* 2018
- **SPINDELBOECK W et al.** Repeated fecal microbiota transplantations attenuate diarrhea and lead to sustained changes in the fecal microbiota in acute, refractory gastrointestinal GVHD. *Haematologica,* 2017
- **TAUR Y et al.** Intestinal Domination and the Risk of Bacteremia in Patients Undergoing Allogeneic Hematopoietic Stem Cell Transplantation. *Clin Infect Dis.,* 2012
- **TAUR Y et al.** The effects of intestinal tract bacterial diversity on mortality following allogeneic hematopoietic stem cell transplantation. *Blood,* 2014
- **VAN LIER et al.** Fecal Microbiota Transplantation Can Cure Steroid-Refractory Intestinal Graft-Versus-Host Disease. *Biology of Blood and Marrow Transplantation,* 2019
- **VOKURKA et al.** Serum citrulline levels as a marker of enterocyte function in patients after allogeneic hematopoietic stem cells transplantation - a pilot study. *Med Sci Monit,* 2013, vol. 19, 81-85
- **WEBER D et al.** Low urinary indoxyl sulfate levels early after transplantation reflect a disrupted microbiome and are associated with poor outcome. *Blood,* 2015
- **YANG Y-W et al.** Use of 16S rRNA Gene-Targeted Group-Specific Primers for Real-Time PCR Analysis of Predominant Bacteria in Mouse Feces. *Appl. Environ. Microbiol.,* October 2015, vol. 81 (19), 6749-6756